# EUROPEAN PATENT APPLICATION

(11) **EP 1 768 199 A1**
(43) Date of publication of application: **28.03.2007**
(21) Application number: 05765489.9
(22) Date of filing: 07.07.2005
(51) Int. Cl.: H01L 51/00

(54) **AROMATIC AMINE DERIVATIVE AND ORGANIC ELECTROLUMINESCENT DEVICE USING SAME**

(30) Priority: 14.07.2004 JP 2004206969
(71) Applicant: IDEMITSU KOSAN CO., LTD., Tokyo 100-8321 (JP)
(72) Inventor: YABUNOUCHI, Nobuhiro, 2990293 (JP); KAWAMURA, Hisayuki, 2990293 (JP); HOSOKAWA, Chishio, 2990293 (JP)
(74) Representative: Gille Hrabal Struck Neidlein Prop Roos
(86) International application number: PCT/JP2005/012606
(87) International publication number: WO 2006/006505

(57) **Abstract**

A novel aromatic amine derivative having an asymmetric structure, and in an organic electroluminescence device which comprises at least one organic thin film layer comprising a light emitting layer sandwiched between a pair of electrode consisting of an anode and a cathode, at least one of the organic thin film layer comprises the aromatic amine derivative singly or as its mixture component. The organic electroluminescence device having an improved success ratio on its production due to difficult cristalization of the amine derivative and exhiviting a longlife time, and also to the aromatic amine derivative for realizing the organic electroluminescence device is provided.

## Description

### TECHNICAL FIELD

The present invention relates to an aromatic amine derivative and an organic electroluminescence ("electroluminescence" will be occasionally referred to as "EL", hereinafter) device employing the aromatic amine derivative, in particular, to an organic EL device having an improved success ratio on its production due to difficult crystallization of the amine derivative and exhibiting a long lifetime, and also to the aromatic amine derivative for realizing the organic EL device; the success ratio means the ratio of the amounts of successfully fabricated device to the total amounts of fabricated device.

### BACKGROUND ART

An organic electroluminescence device is a spontaneous light emitting device which utilizes the principle that a fluorescent substance emits light by energy of recombination of holes injected from an anode and electrons injected from a cathode when an electric field is applied. Since an organic EL device of the laminate type driven under a low electric voltage was reported by C. W Tang et al. of Eastman Kodak Company (C. W. Tang and S. A. Vanslyke, Applied Physics Letters, Volume 51, Pages 913, 1987), many studies have been conducted on organic EL devices using organic materials as the constituting materials. Tang et al. used a laminate structure using tris(8-hydroxyquinolinol aluminum) for the light emitting layer and a triphenyldiamine derivative for the hole transporting layer. Advantages of the laminate structure are that the efficiency of hole injection into the light emitting layer can be increased, that the efficiency of forming excited particles which are formed by blocking and recombining electrons injected from the cathode can be increased, and that excited particles formed among the light emitting layer can be enclosed. As the structure of the organic EL device, a two-layered structure having a hole transporting (injecting) layer and an electron transporting and light emitting layer and a three-layered structure having a hole transporting (injecting) layer, a light emitting layer and an electron transporting (injecting) layer are well known. To increase the efficiency of recombination of injected holes and electrons in the devices of the laminate type, the structure of the device and the process for forming the device have been studied.

Generally, driving or storing an organic EL device under elevated temperature environment causes negative effects such as color shift of light emission, decrease of current efficiency, increase of driving voltage, making short of a light emission lifetime and the like. So as to prevent it from them, it has been required to heighten a glass transition temperature (Tg) of a hole transporting material. Therefore, it has been necessary to have many aromatic groups in the molecule of a hole transportation material (for example, aromatic condensed rings described in the Patent literature 1 and aromatic diamine derivatives described in the Patent literature 2), and a structure having eight to twelve benzene rings has been preferably used.
However, if the molecule has many aromatic groups therein, crystallization tends to take place during production of an organic EL device by forming thin film using the hole transportation materials. Since there have been clogging an outlet of a crucible to be used for vapor deposition and generating defects of the thin layer resulting from the crystallization, there have been problems of decreasing the success ratio on its production and so forth. In addition, although a compound having many aromatic groups in its molecule has generally high glass temperature, there has been a problem of a short lifetime thereof on the ground that a phenomenon such as decomposition of the compound on vapor deposition or uneven vapor deposition due to its high sublimation temperature.
In contrast, there is known literatures disclosing asymmetric aromatic amine derivatives. For example, Patent literature 3 describes an aromatic amine derivative having an asymmetric structure, but dose not provide with not only any specific example thereof but also description about characteristic of an asymmetric compound. In addition, Patent literature 4 describes an asymmetric aromatic amine derivative having a phenanthrene group, but treats it as the same level as a symmetric compound and provides with no description about characteristic of an asymmetric compound. Further, although a specific synthesis method is required to prepare an asymmetric compound, there is no description of any method preparing an asymmetric compound in these patent literatures. More further, Patent literature 5 describes synthesis methods to prepare an asymmetric compound, but provides with no description about characteristic of an asymmetric compound. Patent literature 6 describes asymmetric compounds having high glass temperature and thermal stability, but discloses only an asymmetric compound having a carbazole group as specific example. In addition, the present inventers found the problem that an organic EL device fabricated by using the compound had a short lifetime.
As mentioned above, although organic EL devices having long life times were reported, it having a enough long lifetime has not yet been available. Therefore, development of an organic EL device having more excellent performance has been strongly desired
Patent literature 1: U.S.P 4,720,432
Patent literature 2: U.S.P 5,061,569
Patent literature 3: Japanese Patent Application Laid-open No. Heisei 8 (1996)-48656
Patent literature 4: Japanese Patent Application Laid-open No. Heisei 11 (1999)-135261
Patent literature 5: Japanese Patent Application Laid-open No. 2003-171366
Patent literature 6: U.S.P 6,242,115

### DISCLOSURE OF THE INVENTION

The present invention has been made to overcome the above problems and has an objective of providing an organic EL device exhibiting the improved success ratio on its production due to difficult crystallization of the molecule therein and having a long lifetime, and also providing an aromatic amine derivative for realizing the organic EL device.

As a result of intensive researches and studies to achieve the above objective by the present inventors, it was found that a novel aromatic derivative employing an asymmetric derivative represented by any one of following general formulae from (1) to (3) as a constituting material for an organic EL device particularly as a hole transporting material enables to overcome the above problem. Such being the case, the present invention has been accomplished on the basis of the foregoing findings and information.
Further, it was found that an amino group substituted with an aryl group as an asymmetric amine unit was suitable for the present objective. It was found that the amine unit contributes to improve the success ratio on production of organic EL device on the ground of controlled crystallization which was based on small interaction between the molecules due to steric hindrance thereof. Further, since it could be vapor deposited at low sublimation temperature, it was found that decomposition on vapor deposition was controlled and an organic EL device obtained by using the compound had an advantage of a longer lifetime. In particular, in combination it with a device emitting blue light, it was found that an advantage of an outstanding long lifetime was achieved.

Therefore, the present invention provides an aromatic amine derivative represented by a following general formula (1):

A - L - B (1)

wherein L represents an interbonding group consisting of a substituted or unsubstituted arylene group having 5 to 50 ring atoms, or an interbonding group derived from bonding a plural number of a substituted or unsubstituted arylene group having 5 to 50 ring atoms with a single bond, an oxygen atom, a sulfur atom, a nitrogen atom or a saturated or unsaturated bivalent aliphatic hydrocarbon group having 1 to 20 carbon atoms.

A represents a diarylamino group expressed by a following general formula (2):

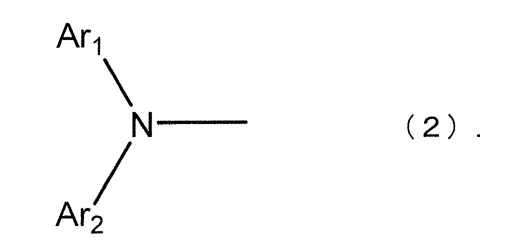

B represents a diarylamino group expressed by a following general formula (3); however, A is not the same as B.

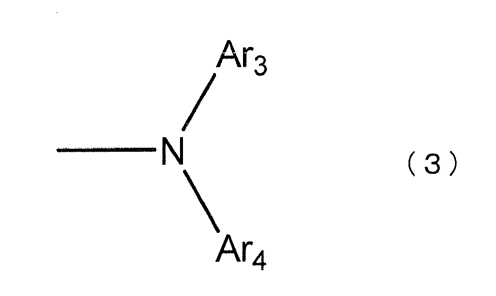

In the above general formulae (2) and (3), Ar₂ to Ar₄ each independently represents a substituted or unsubstituted aryl group having 5 to 50 ring atoms.
In the general formula (2), Ar₁ represents a group expressed by any one of following general formulae (4) to (9):

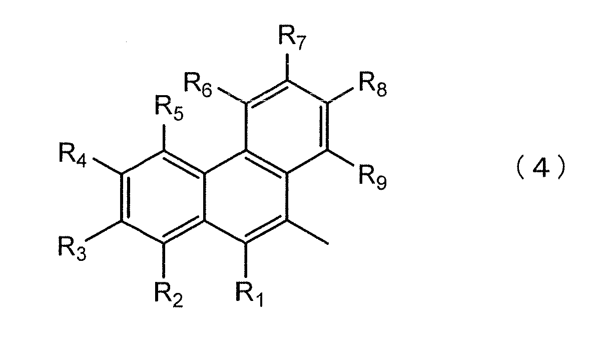

In the general formula (4), R₁ to R₉ each independently represents a hydrogen atom, an aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 ring atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted with a substituted or unsubstituted aryl group having 5 to 50 ring atoms, a halogen atom, a cyano group, a nitro group, a hydroxyl group or a carboxyl group,

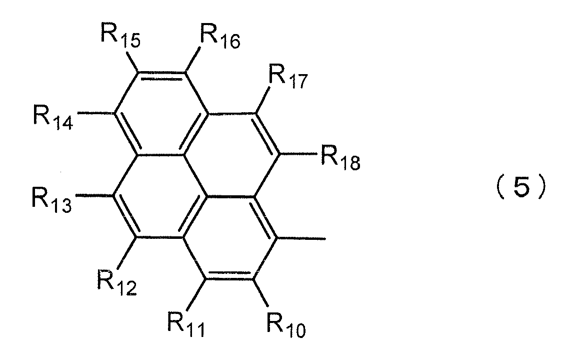

In the general formula (5), R₁₀ to R₁₈ each independently is the same as each of R₁ to R₉ in the general formula (4).

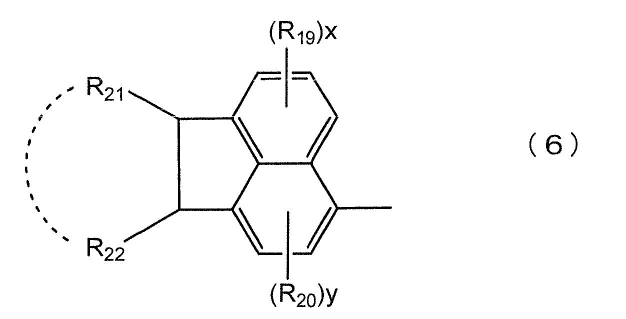

In the general formula (6), R₁₉ to R₂₂ each independently is the same as each of R₁ to R₉ in the general formula (4);
x represents an integer of 0 to 3, and y represents an integer of 0 to 2; R₂₁ and R₂₂ may bond each other to form a ring structure.

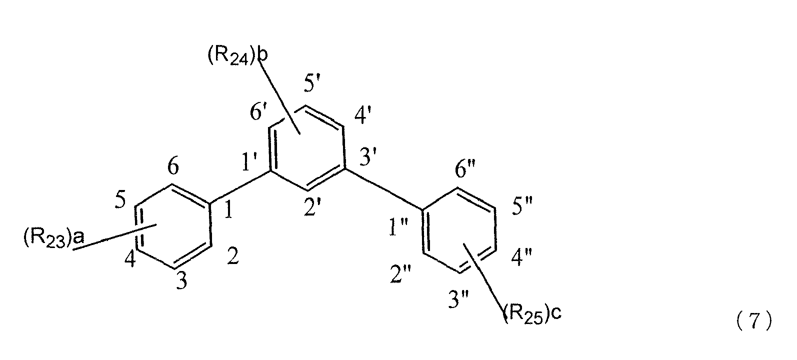

The general formula (7) represents a m-terphenyl group having an interbonding hand obtained by removing a hydrogen atom at any one of 2 to 6, 4' to 6 and 2" to 6" positions ("interbonding hand" means that it bonds with any of R₂₃ to R₂₅ and it means the same as this hereinafter), and R₂₃ to R₂₅ each independently is the same as each R₁ to R₉ in the general formula (4);
a and c each independently represents an integer of 0 to 5, and b represents an integer of 0 to 4,

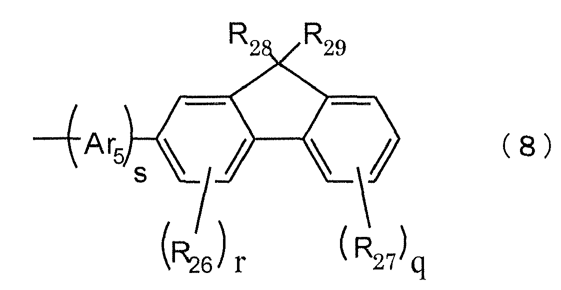

In the general formula (8), Ar₅ represents a substituted or unsubstituted arylene group having 5 to 50 ring atoms or polyarylene group having 5 to 50 ring atoms, or a bivalent group consisting of a substituted or unsubstituted heterocyclic group having 5 to 50 ring atoms or a substituted or unsubstituted diaryl heterocyclic group having 5 to 50 ring atoms; and R₂₆ to R₂₉ each independently is the same as each of R₁ to R₉ in the general formula (4),
s, q and r each independently represents an integer of 0 to 2, R₂₈ and R₂₉ may bond each other to form a ring structure.

Moreover, the present invention provides an organic EL device comprising at least one of organic thin film layers including a light emitting layer sandwiched between a pair of electrodes consisting of an anode and a cathode, wherein the organic thin film layer comprises at least one selected from the aforementioned aromatic amine derivatives singly or as a component of mixture thereof.

The aromatic amine derivatives of the present invention and the derivatives employed for an organic EL device can be hardly crystallized, therefore, the success ratio on production of the device can be improved and also the long lifetime thereof can be achieved.

### THE PREFERRED EMBODIMENT TO CARRY OUT THE INVENTION

The aromatic amine derivatives of the present invention are represented by the general formula (1):

A - L- B (1).

In the general formula (1), L represents an interbonding group consisting of
(I) a substituted or unsubstituted arylene group having 5 to 50 ring atoms, or
(II) an interbonding group derived from bonding a plural number of a substituted or unsubstituted arylene group having 5 to 50 ring atoms with
(II-1) a single bond,
(II-2) an oxygen atom (-O-),
(II-3) a sulfur atom (-S-),
(II-4) a nitrogen atom (-NH-, -NR- wherein R means a substituent) or
(II-5) a saturated or unsaturated bivalent aliphatic hydrocarbon group having 1 to 20 carbon atoms.

An arylene group having 5 to 50 ring atoms in the above items (I) and (II) includes, for example, 1,4-phenylene group, 1,2-phenylene group, 1,3-phenylene group, 1,4-naphthylene group, 2,6-naphthylene group, 1,5-naphthylene group, 9,10-anthranylene group, 9,10-phenanthrenylene group, 3,6-phenanthrenylene group, 1,6-pyrenylene group, 2,7-pyrenylene group, 6,12-chrysenylene group, 1,1'-biphenylene group, 4,4'-biphenylene group, 3,3'-biphenylene group, 2,7-fluorenylene group, 2,5-thiophenylene group, 2,5-silolylene group, 2,5-oxadiazolylene group, terphenylene group and the like. Among those, preferred include 1,4-phenylene group, 1,2-phenylene group, 1,3-phenylene group, 1,4-naphthalene group, 9,10-anthranylene group, 6,12-chrysenylene group, 4,4'-biphenylene group, 3,3-biphenylene group, 2,2-biphenylene group and 2,7-fluorenylene group.

The substituted or unsubstituted bivalent aliphatic hydrocarbon group having 1 to 20 carbon atoms in the above (II-5) may be any one of a linear type, a branch type or a ring type, and includes, for example, methylene group, ethylene group, propylene group, isopropylene group, ethylidene group, cyclohexylidene group, adamantylene group and the like.
L represents preferably phenylene group, biphenylene group, terphenlylene group, fluorenylene group, more preferably biphenylene group, and in particular preferably 1,1'-biphenylene.

In the general formula (1), A represents diarylamino group expressed by a following general formula (2):

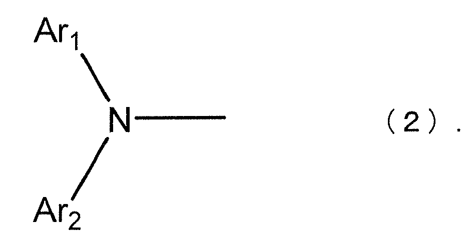

In the general formula (1), B represents diarylamino group expressed by a following general formula (3):

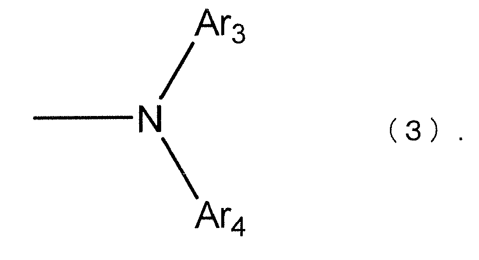

However, A and B are not the same with each other in the general formula (1).
Ar₂ to Ar₄ in the general formulae (2) and (3) each independently represents a substituted or unsubstituted aryl group having 5 to 50 ring atoms.

Examples of the aryl group represented by Ar₂ to Ar₄ include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl) phenyl group, 3-methyl-2-naphthyl group, 4-methyl-1-naphthyl group, 4-methyl-1-anthryl group, 4'-methylbiphenyl-yl group, 4"-t- butyl-p-terphenyl-4-yl group, fluoranthenyl group, fluorenyl group, 1-pyrrolyl group, 2-pyrrolyl group, 3-pyrrolyl group, pyradinyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxanyl group, 5-quinoxanyl group, 6-quinoxanyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,1.0-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrohn-10-yl group, 1-phenazinyl group, 2-phenazinyl group, 1-phenothiazinyl group, 2-phenothiazinyl group, 3-phenothiazinyl group, 4-phenothiazinyl group, 10-phenothiazinyl group, 1-phenoxazinyl group, 2-phenoxazinyl group, 3-phenoxazinyl group, 4-phenoxazinyl group, 10-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrole-5-yl group, 3-methylpyrrole-1-yl group, 3- methylpyrrole-2-yl group, 3-methylpyrrole-4-yl group, 3- methylpyrrole-5-yl group, 2-t-butylpyrrole-4-yl group, 3-(2-phenylpropyl)pyrrole-1-yl group, 2-carbinyl-1 indolyl group, 4- carbinyl-1-indolyl group, 2-carbinyl-3-indolyl group, 4-carbinyl-3-indolyl group, 2-t-butyl 1-indolyl group, 4-t-butyl 1-indolyl group, 2-t-butyl 3-indolyl group, 4-t-butyl 3-indolyl group and the like.
Among those, phenyl group, naphthyl group, biphenyl group, anthranyl group, phenathryl group, pyrenyl group, chrysenyl group, fluoranthenyl group and fluorenyl group are preferable.

Ar₁ in the general formula (2) represents a group expressed by any one of following general formulae (4) to (9).

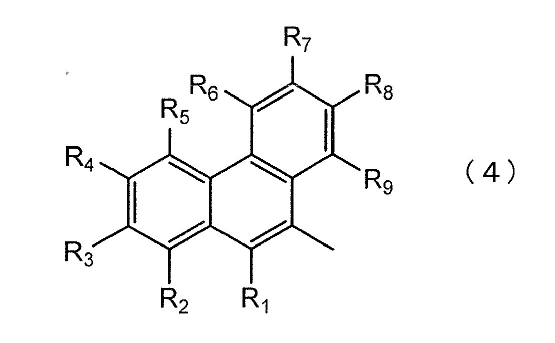

In the general formula (4), R₁ to R₉ each independently represents a hydrogen atom, an aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 ring atoms, a substituted or unsubstituted aryloxy group having 5 to 50 ring atoms, a substituted or unsubstituted arylthio group having 5 to 50 ring atoms, an amino group substituted with a substituted or unsubstituted aryl group having 5 to 50 ring atoms, a halogen atom, a cyano group, a nitro group, a hydroxyl group or a carboxyl group.

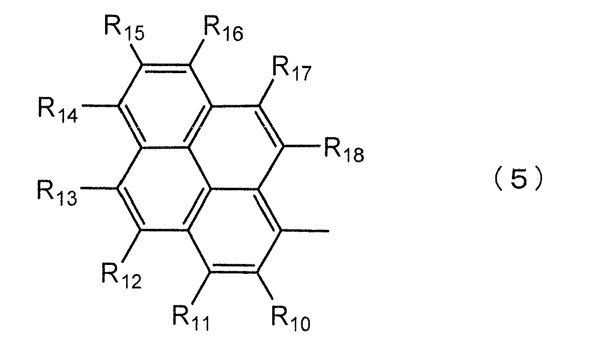

R₁₀ to R₁₈ in the general formula (5) each independently represents the same as each of R₁ to R₉ in the general formula (4).

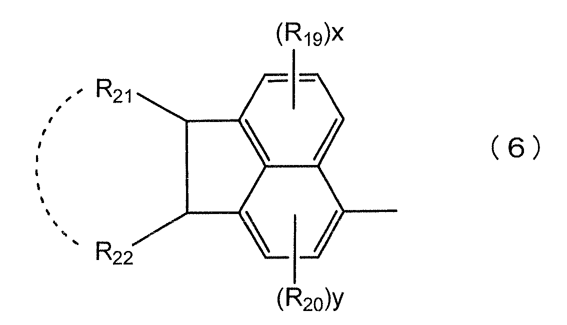

In the general formula (6), R₁₉ to R₂₂ each independently represents the same as each of R₁ to R₉ in the general formula (4);
x represents an integer of 0 to 3 and y represents an integer of 0 to 2; and further, R₂₁ and R₂₂ may bond each other to form a ring structure.
The ring structure, which may be formed by bonding R₂₁ with R₂₂ each other in the general formula (6), includes, for example, cycloalkane having 4 to 12 carbon atoms such as cyclobutane, cylopentane, cyclohexane, adamantane and norbornane, cycloalkene having 4 to 12 carbon atoms such as cyclobutene, cyclopentene, cyclohexene, cycloheptene and cyclooctene, cycloalkadiene having 6 to 12 carbon atoms such as cyclohexadiene, cycloheptadiene and cyclooctadiene, and aromatic ring having 6 to 50 carbon atoms such as benzene, naphtharene, phenanthrene, anthracene, pyrene, chrysene and acenaphthylene.

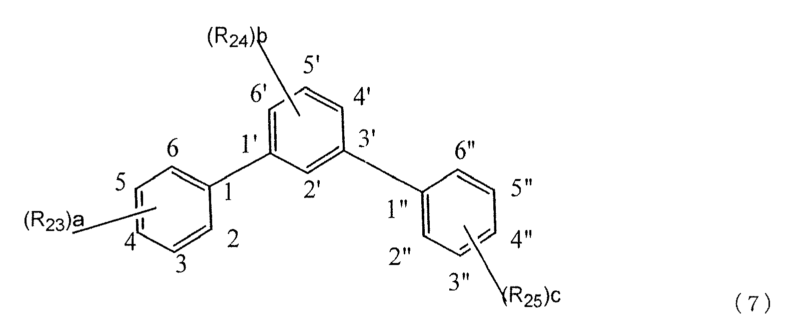

The general formula (7) represents a m-terphenyl group having the interbonding hand obtained by removing a hydrogen atom from any one of 2 to 6, 4' to 6 and 2" to 6" positions, and R₂₃ to R₂₅ each independently is the same as each R₁ to R₉ in the general formula (4);
a and c in general formula (7) each represents an integer of 0 to 5 and b represents an integer of 0 to 4.
A ring position of the interbonding hand, which bonds to a N atom, at the m-terphenyl group is not limited, however, a m-terphenyl group having the interbonding hand at a ring position of 3, 4, 5, 2' or 5' is easily obtained. Further, a m-terphenyl group having the interbonding hand at the ring position of 4 represented by the general formula (7') is particularly suitable to produce an amine compound of the present invention. In addition, the m-terphenyl group may have substituent as aforementioned.

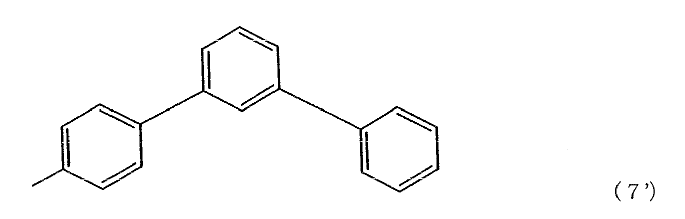

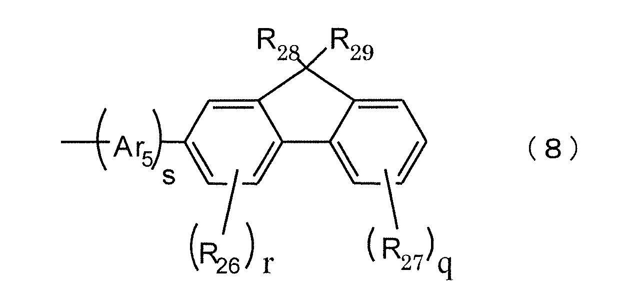

In the general formula (8), Ar₅ represents a substituted or unsubstituted arylene group having 5 to 50 ring atoms or polyarylene group having 5 to 50 ring atoms, or a bivalent group consisting of a substituted or unsubstituted heterocyclic group having 5 to 50 ring atoms or a substituted or unsubstituted diaryl heterocyclic group having 5 to 50 ring atoms; and R₂₆ to R₂₉ each independently is the same as each R₁ to R₉ in the general formula (4);
s, q and r each independently represents an integer of 0 to 2, and R₂₈ and R₂₉ may bond each other to form a ring structure.

An arylene group and a polyarylene group of Ar₅ in the general formula (8) includes, for example, 1,4-phenylene group, 1,2-phenylene group, 1,3-phenylene group, 1,4-naphthylene group, 2,6-naphthylene group, 1,5-naphthylene group, 9,10-anthranylene group, 9,10-phenanthrenylene group, 3,6-phenanthrenylene group, 1,6-pyrenylene group, 2,7-pyrenylene group, 6,12-chrysenylene group, 4,4'-biphenylene group, 3,3'-biphenylene group, 2,2-biphenylene group, 2,7-fluorenylene group, 2,5-thiophenylene group, 2,5-silolylene group, 2,5-oxadiazolylene group and the like. Among those, preferred includes 1,4-phenylene group, 1,2-phenylene group, 1,3-phenylene group, 1,4-naphthylene group, 9,10-anthranylene group, 6,12-chrysenylene group, 4,4'-biphenylene group, 3,3'-biphenylene group, 2,2-biphenylene group, 2,7-fluorenylene group and the like.

A heterocyclic group and a diaryl heterocyclic group as Ar₅ in the general formula (8) includes pyridyl group, pyradinyl group, pyrimidinyl group, pyridazinyl group, triazinyl group, indolin yl group, quinolinyl group, acridinyl group, pyrrolidinyl group, dioxanyl group, piperidinyl, morpholidinyl group, piperazinyl group, triathinyl group, carbazolyl group, furanyl group, thiophenyl group, oxazolyl group, oxadiazolyl group, benzooxazolyl group, thiaoxazolyl group, thiadiazolyl group, imidazolyl group, pranyl group and the like.
An example of a ring structure which may be formed by R₂₈ and R₂₉ in the general formula (8) includes the similar ones explained in the general formula (6).

An example of a substituted or unsubstituted aryl group having 5 to 50 ring atoms represented R₁ to R₂₉ in the general formulae (4) to (8) includes the similar ones explained about aforementioned Ar₂ to Ar₄.
Examples of the substituted or unsubstituted alkyl group having 1 to 50 carbon atoms represented by R₁ to R₂₉ include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxy-isopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyano-propyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group, cyclopropyl group, cyclobutyl group, cyclopentyl group, cyclohexyl group, 4-methylcyclohexyl group, 1-adamanthyl group, 2-adamanthyl group, 1-norbornyl group, 2-norbornyl group and the like.

A substituted or unsubstituted alkoxy group having 1 to 50 carbon atoms represented by R₁ to R₂₉ means a group expressed by -OY and an example of Y includes the similar ones explained in the above alkyl group.
Examples of a substituted or unsubstituted aralkyl group having 6 to 50 ring atoms represented by R₁ to R₂₉ include benzyl group, 1-phenylethyl group, 2-phenylethyl group, 1-phenyl-isopropyl group, 2-phenylisopropyl group, phenyl-t-butyl group, α -naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, 2-β-naphthylisopropyl group, 1-pyrrolylmethyl group, 2-(1-pyrrolyl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group, 1-chloro-2-phenylisopropyl group and the like.

The substituted or unsubstituted aryloxy group having 5 to 50 ring atoms represented by R₁ to R₂₉ means a group expressed by -OY' and an example of Y' includes the similar aryl groups explained in Ar₂ to Ar₄.
The substituted or unsubstituted arylthio group having 5 to 50 ring atoms represented by R₁ to R₂₉ means a group expressed by -SY and an example of Y includes the similar aryl groups explained in Ar₂ to Ar₄.
The substituted or unsubstituted alkoxycarbonyl group having 1 to 50 carbon atoms represented by R₁ to R₂₉ means a group expressed by - COOY and an example of Y includes the similar ones explained in aforementioned alkyl group.
Examples of the aryl group in the an amino group substituted by a substituted or unsubstituted aryl group having 5 to 50 ring atoms represented by R₁ to R₂₉ include the similar aryl groups explained in Ar₂ to Ar₄.
Examples of the halogen atom represented by R₁ to R₂₉ include fluorine atom, chlorine atom, bromine atom and the like.

It is preferable that Ar₁ of the amine derivatives of the present invention is different from any one of Arz to Ar₄ in the general formula (1).
Further, it is preferable that at least one of Ar₂ to Ar₄ is a substituted or unsubstituted condensed ring having 10 to 50 ring atoms in the general formula (1).
An example of the substituted or unsubstituted condensed ring having 10 to 50 ring atoms includes the condensed rings in the aryl groups explained in above Ar₂ to Ar₄.
As the aromatic amine derivatives of the present invention, it is preferable that the B in the general formula (1) corresponds to a diarylamino group expressed by a following general formula (9), and it is more preferable that the B corresponds to a diarylamino group expressed by a following general formula (10).

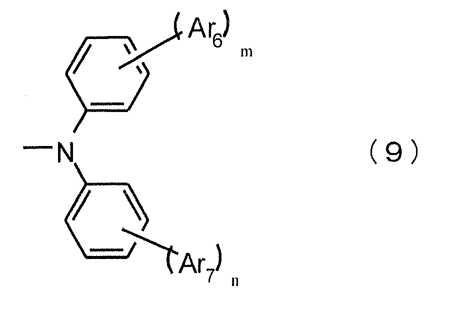

In the general formula (9), Ar₆ and Ar₇ each independently represents a substituted or unsubstituted aryl group having 5 to 50 ring atoms, and an example of the aryl group includes the similar aryl groups explained in the above Ar₂ to Ar₄; m represents an integer of 1 to 5 and n represents an integer of 0 to 5.

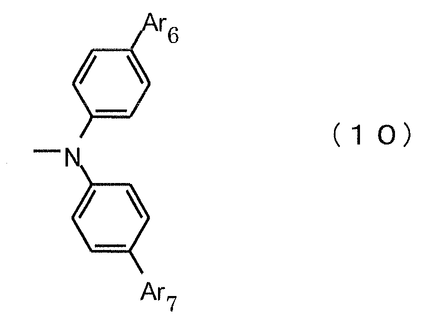

In the general formula (10), Ar₆ and Ar₇ each is the same as the aforementioned.

A substituent of aforementioned Ar₁ to Ar₇, R₁ to R₂₉ and L includes a substituted or unsubstituted aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 1 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted by a substituted or unsubstituted aryl group having 5 to 50 ring atoms, a halogen atom, a cyano group, a nitro group, a hydroxyl group, a carboxyl group and the like.

The amine derivatives of the present invention are preferable for a material of an organic EL device and are more preferable for a hole transporting material of an organic device.
A specific example of the aromatic amine derivatives of the present invention is shown as follows, though not limited thereto.

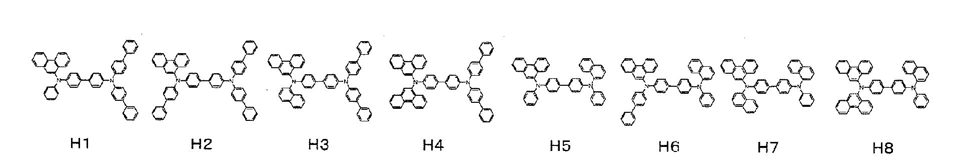

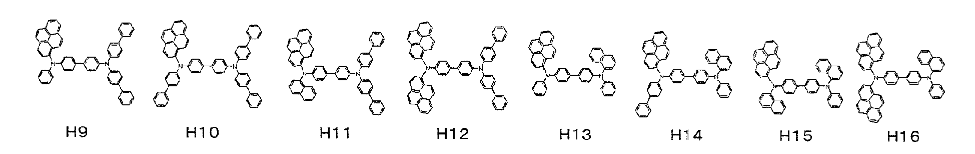

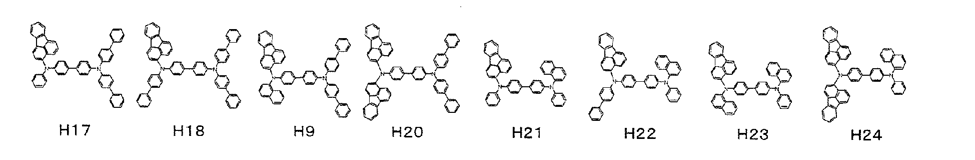

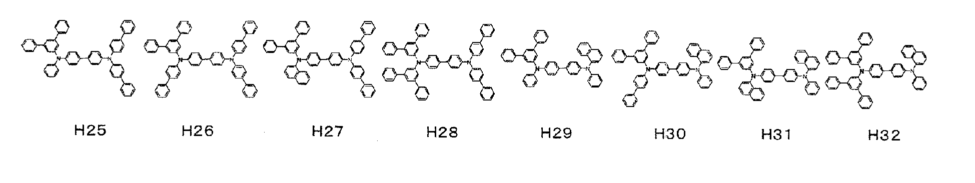

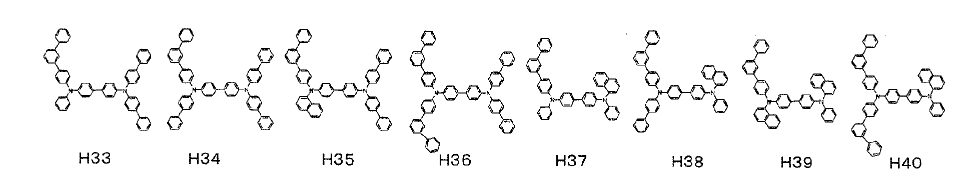

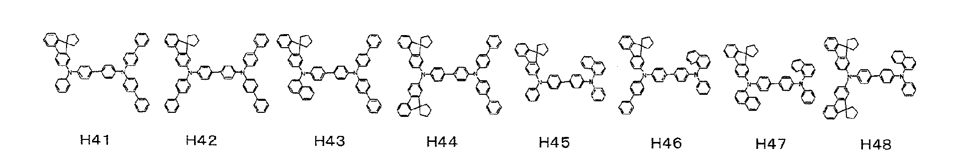

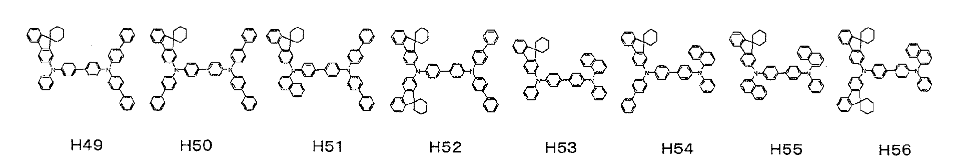

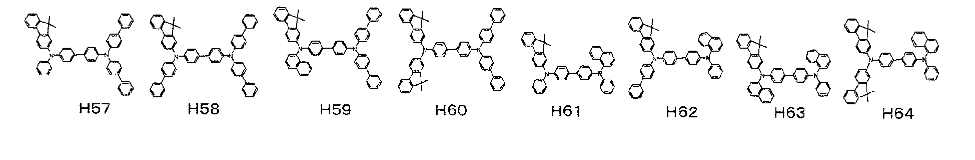

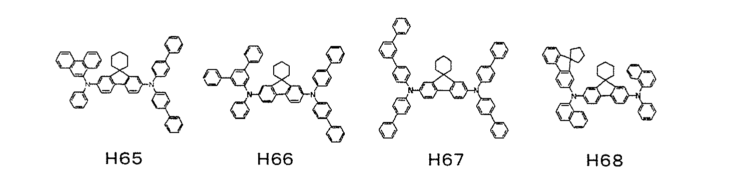

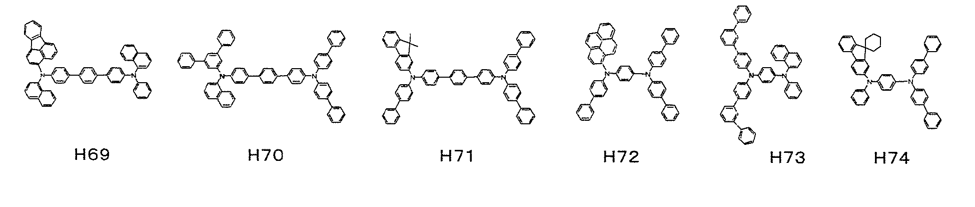

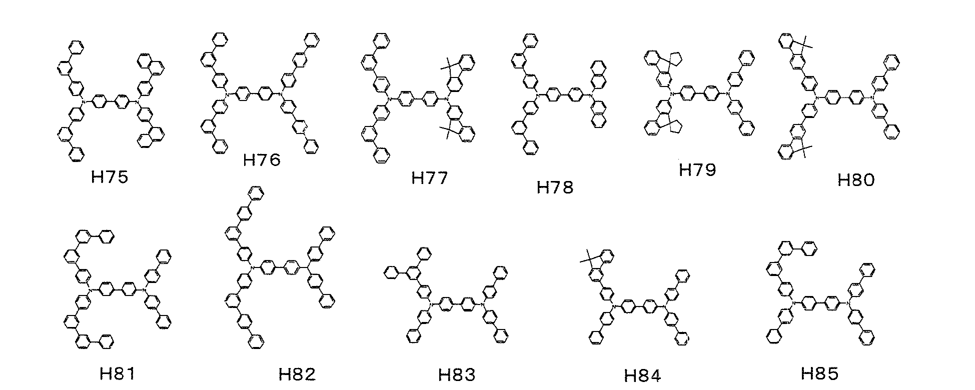

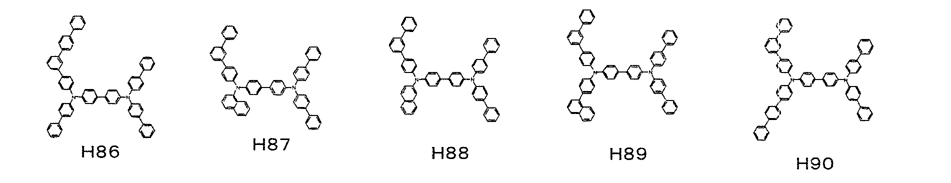

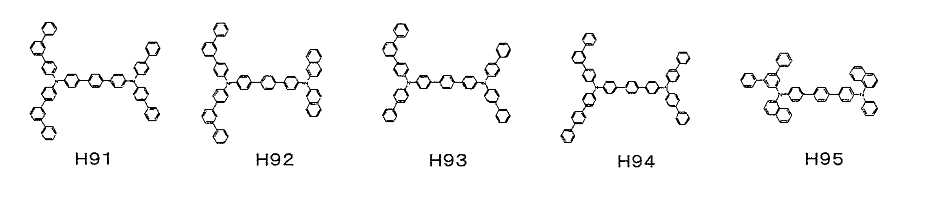

Following is a description regarding a device structure about the organic EL device of the present invention.
The present invention provides an organic EL device which comprises at least one organic thin film layer sandwiched between a pair of electrodes consisting of an anode and a cathode, wherein the organic thin film layers comprises the aforementioned aromatic amine derivative singly or in combination thereof.
It is preferable that an organic EL device of the present invention comprises a hole transporting layer of the above organic thin film layer and the hole transporting layer comprises the aromatic amine derivative or as a component of a mixture: Further, it is preferable that the hole transporting layer comprises the aromatic amine derivative as an essential component.
In particular, it is preferable that the aromatic amine derivative is employed for an organic EL device emitting bluish light.

Further, it is preferable that the organic EL device comprises a light emitting layer containing an aryl amine compound and/or a styryl amine compound.
The aryl amine compound includes compounds represented by a following general formula (A), and the styryl amine compound includes compounds represented by a following general formula (B).

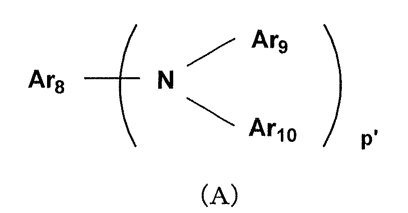

In the general formula (A), Ar₈ represents a group selected from the group of phenyl group, biphenyl group, terphenyl group, stilbene group and distilbene group. Ar₉ and Ar₁₀ each represent a hydrogen atom or an aromatic group having 6 to 20 carbon atoms, and Ar₉ and Ar₁₀ may be substituted. p' represents an integer of 1 to 4. Further, a styryl group in Ar₉ and/or Ar₁₀ is substituted.
Here, the aromatic group having 6 to 20 carbon atoms includes preferably a phenyl group, a naphthyl group, an anthranyl group, a phenanthryl group, a terphenyl group and the like.

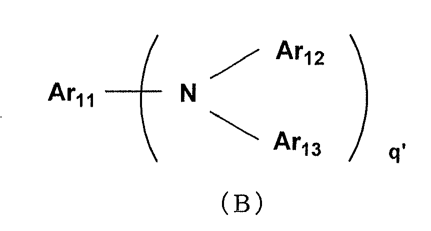

In the general formula (B), Ar₁₁ to Ar₁₃ represents an aryl group having 5 to 40 ring atoms, which may be substituted; q' represents an integer of 1 to 4.
Here, a preferable aryl group having 5 to 40 ring atoms includes phenyl, naphthyl, anthranyl, phenanthryl, pyrenyl, coronyl, biphenyl, terphenyl, pyrrolyl, furanyl, thiophenyl, benzothiophenyl, oxadiazolyl, diphenylanthranyl, diphenylanthranyl, indryl, carbazolyl, pyridyl, benzoquinolyl, fluolanthenyl, acetofluolanthenyl, stilbene and the like. In addition, the aryl group having 5 to 40 ring atoms may be further substituted by substituent. The preferable substituent includes an alkyl group having 1 to 6 carbon atoms such as ethyl group, methyl group, i-propyl group, n-propyl group, s-butyl group, t-butyl group, pentyl group, hexyl group, cyclopentyl group, cyclohexyl group and the like, an alkoxy group having 1 to 6 carbon atoms such as ethoxy group, methoxy group, i-propoxy group, n-propoxy group, s-butoxy group, t-butoxy group, pentoxy group, hexyloxy group, cyclopentoxy group, cyclohexyloxy group and the like, an aryl group having 5 to 40 ring atoms, an amino group substituted by an aryl group having 5 to 40 ring atoms, an ester group having an aryl group having 5 to 40 ring atoms, an ester group having an alkyl group having 1 to 6 carbon atoms, a cyano group, a nitro group and a halogen atom such as chlorine, bromine, iodine and etc.

The construction of the organic EL device of the present invention will be explained as follows:

### (1) The construction of the organic EL device

Typical examples of the construction of the organic EL device of the present invention include:
(1) An anode / a light emitting layer / a cathode;
(2) An anode / a hole injecting layer / a light emitting layer / a cathode;
(3) An anode / a light emitting layer / an electron injecting layer / a cathode;
(4) An anode / a hole injecting layer / a light emitting layer / an electron injecting layer / a cathode;
(5) An anode / an organic semiconductor layer / a light emitting layer / a cathode;
(6) An anode / an organic semiconductor layer / an electron barrier layer / a light emitting layer / a cathode;
(7) An anode / an organic semiconductor layer / a light emitting layer / an adhesion improving layer / a cathode;
(8) An anode / a hole injecting layer / a hole transporting layer / a light emitting layer / an electron injecting layer / a cathode;
(9) An anode / an insulating layer / a light emitting layer / an insulating layer / a cathode;
(10) An anode /an inorganic semiconductor layer / an insulating layer / a light emitting layer /an insulating layer / a cathode;
(11) An anode / an organic semiconductor layer / an insulating layer / a light emitting layer / an insulating layer / a cathode;
(12) an anode / an insulating layer / a hole injecting layer / a hole transporting layer / a light emitting layer / an insulating layer / a cathode; and
(13) An anode / an insulating layer / a hole injecting layer / a hole transporting layer /a light emitting layer / an electron injecting layer / a cathode.
Among those, the construction (8) is generally employed in particular; however, the construction of the organic EL device is not limited to those shown above as the examples.
Although the aromatic amine derivatives of the present invention may be employed for any of the above organic layers, it is preferable that it is contained in a light emitting zone or a hole transporting zone among those construction elements. The aforementioned success ratio is improved when it is employed preferably in a light emitting zone or a hole transporting zone, more preferably in a hole transporting zone, in particular preferably in a hole transporting layer.
An amount of the amine derivatives to be contained in above organic layers is preferably in the range of from 30 to 100 mole%.

### (2) Substrate which transmits light

The organic EL device is fabricated on a substrate which transmits light. The substrate which transmits light has a role of supporting an organic EL device. It is preferable that it has a transmittance of light of 50% or greater in the visible region of 400 to 700 nm as well as flat and smooth thereof.
As the substrate which transmits light, for example, glass sheet and synthetic resin sheet are advantageously employed. Specific examples of the glass sheet include soda ash glass, glass containing barium and strontium, lead glass, aluminosilicate glass, borosilicate glass, barium borosilicate glass and quartz. Specific examples of the synthetic resin sheet include sheet made of polycarbonate resins, acrylic resins, polyethylene terephthalate resins, polyether sulfide resins and polysulfone resins.

### (3) Anode

The anode in the organic EL device of the present invention covers a role of injecting holes into a hole transport layer or into a light emitting layer, and it is effective that the anode has a work function of 4.5 eV or greater. Specific examples of the material for the anode include indium tin oxide (ITO) alloy, tin oxide (NESA), gold, silver, platinum, copper, etc.
The anode can be prepared by forming a thin film of the electrode material described above in accordance with a process such as a vapor deposition process or a sputtering process.
When the light emitted from the light emitting layer is observed through the anode, it is preferable that the anode has a transmittance of the emitted light greater than 10 %. It is also preferable that the sheet resistivity of the anode is several hundred Ω /□ or smaller. The thickness of the anode is, in general, selected in the range of from 10 nm to 1µ m and preferably in the range of from 10 to 200 nm.

### (4) Light emitting layer

In the organic EL device of the present invention, the light emitting layer has the following functions:
(1) The injecting function: the function of injecting holes from the anode or the hole injecting layer and injecting electrons from the cathode or the electron injecting layer when an electric field is applied;
(2) The transporting function: the function of transporting injected charges (electrons and holes) by the force of the electric field; and
(3) The light emitting function: the function of providing the field for recombination of electrons and holes and leading the recombination to the emission of light.
Although there may be a difference between the capability of the holes being injected and the capability of the electrons being injected, and although there may be a grade about the transporting function expressed by mobility of the holes and the electrons, it is preferable to move charges of either ones.
As the process for forming the light emitting layer, a well known process such as the vapor deposition process, the spin coating process and the LB process can be employed. It is preferable that a light emitting layer is a molecular sedimentation film particularly. Here, the molecular sedimentation film is defined as a thin film formed by sedimentation of a gas phase material compound or a thin film formed by condensation of a liquid phase material compound. The molecular sedimentation film may be differentiated from a thin film (a molecular build-up film) formed by the LB process, base on the differences between agglomeration structures and higher-order structures, and also the differences resulting from functionalities thereof.
In addition, as shown in Japanese Patent Laid-open No. Showa57(1982)-51781, to form a light emitting layer, a thin film may be formed in accordance with the spin coating process and the like of the solution to be prepared by dissolving a binder such as resin and a material compound in solvent.
In the present invention, any well known light emitting material other than a light emitting material consisting of an asymmetric pyrene derivative of the present invention may be optionally contained in the light emitting layer; or a light emitting layer containing other well known light emitting layer may be laminated with the light emitting layer containing the light emitting material of the present invention each in an extent of not obstructing to achieve the objective of the present invention respectively.

A light emitting material or a dopant to be used together with the aromatic amine derivatives includes, for example, anthracene, naphthalene, phenanthrene, pyrene, tetracene, coronene, chrysene, fluorescein, perylene, phthaloperylene, naphtaloperylene, perinone, phthaloperinone, naphthaperinone, diphenylbutadiene, tetraphenylbutadiene, coumarine, oxadiazole, aldazine, bisbenzooxazoline, bisstyryl, pyrazine, cyclopentadiene, quinolin metal complex, aminoquinolin metal complex, benzoquinolin metal complex, imine, diphenylethylene, vinylanthracene, diaminecarbazol, pyran, thiopyran, polymethyne, merocyanine, imidazol chelate oxinoid compound, quinacridone, rubrene and fluorescent dye, but not limited thereto.

A preferable host material to be used together with the aromatic amine derivatives of the present invention includes compounds represented by following general formulae (i) to (ix).
An asymmetric anthracene represented by a following general formula (i): 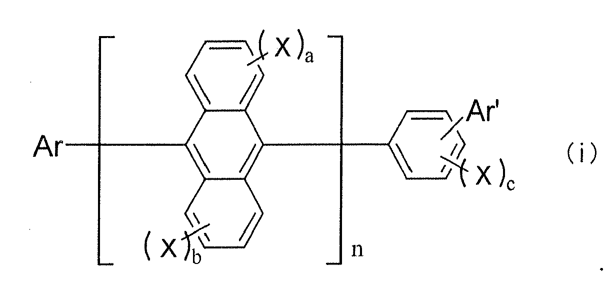 In the above general formula (i), Ar represents a substituted or unsubstituted condensed aromatic group having 10 to 50 ring carbon atoms; Ar' represents a substituted or unsubstituted aromatic group having 6 to 50 ring carbon atoms;
X represents a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 6 to 50 ring atoms, a substituted or unsubstituted arylthio group having 5 to 50 ring atoms, a substituted or unsubstituted alkoxycarbonyl group having 1 to 50 carbon atoms, a carboxyl group, a halogen atom, a cyano group, a nitro group and a hydroxyl group;
a, b and c each independently represents an integer of 0 to 4;
n represents an integer of 1 to 3, and further, a case where n represents 2 or greater, the plural group within "[ ]" may be the same with or different from each other.

An asymmetric mono-anthracene derivative represented by a following general formula (ii): 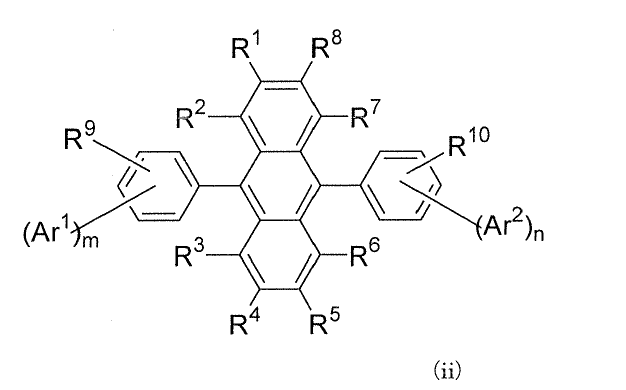 In the general formula (ii), Ar¹ and Ar² each independently represents a substituted or unsubstituted aromatic ring group having 6 to 50 ring carbon atoms; m and n each represents an integer of 1 to 4; however, in a case where m=n=1 and at the same time, where each bonding position of Ar¹ and Ar² to a benzene ring is monosymmetric each other, Ar¹ is different from Ar², and in a case where m or n represents an integer of 2 to 4, m is different from n;
R¹ to R¹⁰ each independently represents a hydrogen atom, a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group and a hydroxyl group.

An asymmetric pyrene derivative represented by a following general formula (iii): 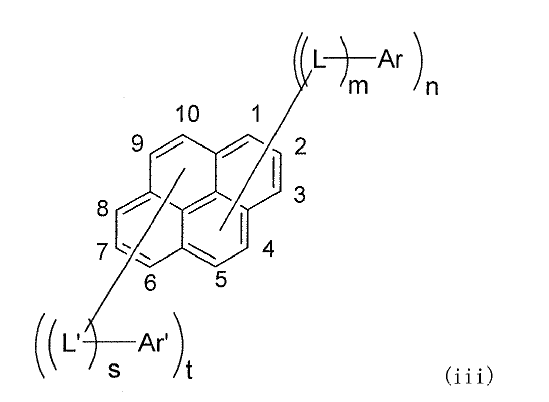 In the general formula (iii), Ar and Ar' each independently a substituted or unsubstituted aromatic group having 6 to 50 ring carbon atoms;
L and L' each independently represents a substituted or unsubstituted phenylene group, a substituted or unsubstituted naphthalene group, a substituted or unsubstituted fluorenylene group or a substituted or unsubstituted dibenzosilolylene group;
m represents an integer of 0 to 2, n represents of an integer of 1 to 4, s represents an integer of 0 to 2 and t represents an integer of 0 to 4;
further, L or Ar bonds to any one of 1 to 5 position of pyrene, and L' or Ar' bonds to any one of 6 to 10 position thereof; however, when n + t is an even number, Ar, Ar', L and L' satisfy a following requirement (1) or a requirement (2):
(1) Ar ≠ Ar' and/or L ≠ L' (wherein ≠ means that each group has a different structure)
(2) when Ar = Ar' and L = L'
   (2-1) m ≠ s and/or n ≠ t, or
   (2-2) when m = s and n = t,
      (2-2-1) both L and L' or pyrene bond respectively to a different position of Ar and Ar', or
      (2-2-2) both L and L' or pyrene bond respectively to the same position of Ar and Ar' excluding a case where both L and L' or both Ar and Ar' bond respectively to 1 and 6, or 2 and 7 positions thereof.

An asymmetric anthracene derivative represented by a following general formula (iv): 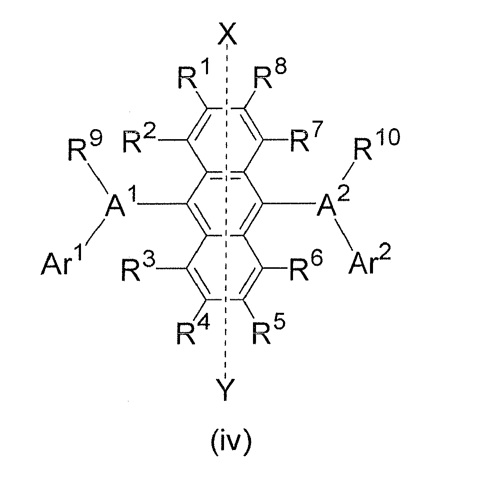 In the general formula (iv), A¹ and A² each independently represents a substituted or unsubstituted condensed aromatic group having 10 to 20 ring carbon atoms;
Ar¹ and Ar² each independently represents a hydrogen atom, a substituted or unsubstituted aromatic group having 6 to 50 ring carbon atoms,
R¹ to R¹⁰ each independently represents a hydrogen atom, a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group or a hydroxyl group.
Ar¹, Ar², R⁹ and R¹⁰ each may be more than one, and two neighboring groups thereof may form a saturated or unsaturated ring structure, however, a case where the groups at 9 and 10 positions of anthracene at the core are symmetrical about X-Y axis of symmetry and bond each other is excluded.

An anthracene derivative represented by a following general formula (v): 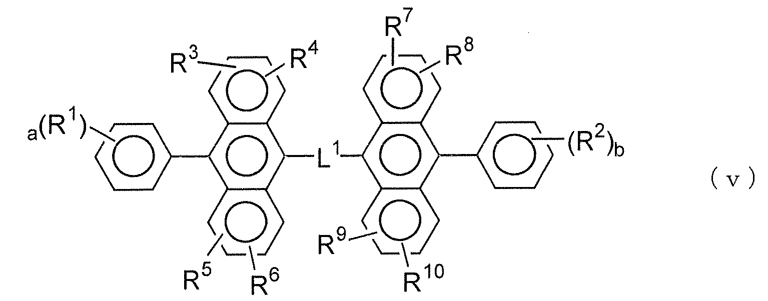 In the general formula (v), R¹ to R¹⁰ each independently represents a hydrogen atom, an alkyl group, a cycloalkyl group, an aryl group which may be substituted, an alkoxyl group, an aryloxy group, an alkylamino group, an alkenyl group, an arylamino group or a heterocyclic group which may be substituted; a and b each represents an integer of 1 to 5, and when both of a and b are 2 or greater, both R¹ or both R² may be the same with or different from each other, additionally both R¹ or both R² may bond each other to form a ring; both R³ and R⁴, both R⁵ and R⁶, both R⁷ and R⁸, and/or both R⁹ and R¹⁰ may bond each other to form a ring, L¹ represents a single bond, -O-, -S-, -N-(R)-, an alkylene group or an arylene; wherein R represents an alkyl group, or an aryl group which may be substituted.

An anthracene derivative represented by a following general formula (vi): 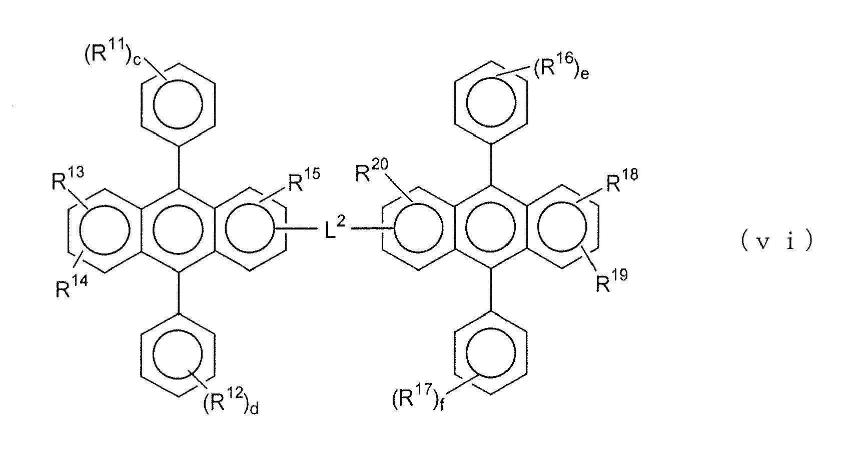 In the general formula (vi), R¹¹ to R²⁰ each independently represents a hydrogen atom, an alkyl group, a cycloalkyl group, an aryl group, an alkoxyl group, an aryloxy group, an alkylamino group, an arylamino group or a heterocyclic group which may be substituted; c, d, e and f each represents an integer of 1 to 5, and when c, d, e and/or f are 2 or greater, plural R¹¹, plural R¹², plural R¹⁶ or plural R¹⁷ may be the same with or different from each other, additionally plural R¹¹, plural R¹², plural R¹⁶ or plural R¹⁷ may bond each other to form a ring; both R¹³ and R¹⁴, and/or both R¹⁸ and R¹⁹ may bond each other to form a ring; L² represents a single bond, -O-, -S-, -N-(R)-, an alkylene group or an arylene; wherein R represents an alkyl group, or an aryl group which may be substituted.

A spirofluorene derivative represented by a following general formula (vii): 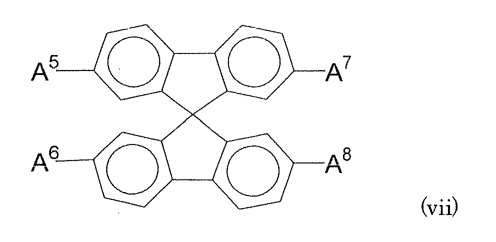 In the general formula (vii), A⁵ to A⁸ each independently represented a substituted or unsubstituted biphenyl group or a substituted or unsubstituted naphthyl group.

A compound containing a condensed ring represented by a following general formula (viii): 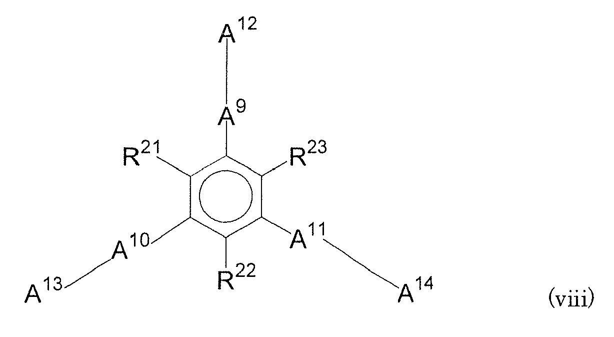 In the general formula (viii), A⁹ to A¹⁴ each independently represents the same as aforementioned, and R²¹ to R²³ each independently represents a hydrogen atom, an alkyl group having 1 to 6 carbon atoms, a cycloalkyl group having 3 to 6 carbon atoms, an alkoxyl group having 1 to 6 carbon atoms, an aryloxy group having 5 to 18 carbon atoms, an aralkyloxy group having 7 to 18 carbon atoms, an arylamino group having 5 to 16 carbon atoms, a nitro group, a cyano group, an ester group having 1 to 6 carbon atoms or a halogen atom, and at least one of A⁹ to A¹⁴ represents a condensed aromatic ring comprising 3 or more rings.

A fluorene compound represented by a following general formula (ix): 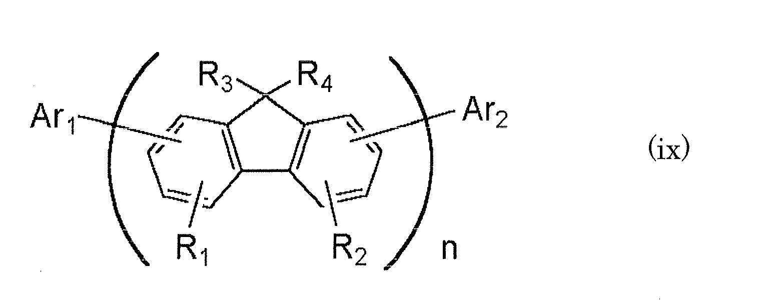 In the general formula (ix), R₁ and R₂ each independently represents a hydrogen atom, a substituted or unsubstituted alkyl group, a substituted or unsubstituted aralkyl group, a substituted or unsubstituted aryl group, a substituted or unsubstituted heterocyclic group, a substituted amino group, a cyano group or a halogen atom; both R₁ and both R₂ bonding to a different fluorene group may be the same with or different from each other, and both R₁ and R₂ bonding to the same fluorene group may be the same with or different from each other; R₃ and R₄ each independently represents a hydrogen atom, a substituted or unsubstituted alkyl group, a substituted or unsubstituted aralkyl group, a substituted or unsubstituted aryl group, or a substituted or unsubstituted heterocyclic group, and both R₃ and both R₄ bonding to a different fluorene group may be the same with or different from each other, and also both R₃ and R₄ bonding to the same fluorene group may be the same with or different from each other; Ar₁ and Ar₂ each independently represents a substituted or unsubstituted condensed polycyclic aromatic group consisting of benzene rings of 3 or more or a substituted or unsubstituted condensed polycyclic heterocyclic group comprising of benzene rings and hetero rings of 3 or more in total. Ar₁ and Ar₂ may be the same with or different from each other, n represents an integer of 1 to 10.

Among the above host materials, an anthracene derivative is preferable and a monoanthracene derivative is more preferable, further an asymmetric anthracene is particularly preferable.
In addition, a phosphorescent compound may be employed as a light emitting material for dopant. A compound containing a carbazole ring for a host material is preferable as a phosphorescent compound. Although a dopant is a compound which is able to emit light from triplet exciton and is not limited as long as emitting light from triplet exciton, it is preferable that a metal complex contains at least a metal selected from a group consisting of Ir, Ru, Pd, Pt, Os and Re. A porphyrin metal complex or an orthometallized metal complex is preferable.
A suitable host for phosphorescence comprising a compound containing a carbazole ring is a compound having a function of making a phosphorescent compound to emit light as a result of energy transfer from its excitation state to the phosphorescent compound. With regard to the host compound, any compound being able to transfer exciton energy to the phosphorescent compound may be selected, without particularly restricted, for the purpose as appropriate. Any heteroring excluding a carbazole ring may be contained.

Specific examples of the host compound include a carbazole derivative, a triazole derivative, an oxazole derivative, an imidazole derivative, a polyarylalkane detivative,a pyrazoline derivative, a pyrazlone derivative, a phenylene diamine derivative, an aryamine derivative, a calcone derivative substituted by amine, a atyrylanthracene derivative, a fluorene derivative, hydrazone derivative, a stilbene derivative, a silazane derivative, an aromatic tertiary amine compound, a styrylamine compound, an aromatic dimethylidene type compound, a porphyrin type compound, an anthtaquinone dimethane derivative, a diphenylqyinone derivative, a thiopyran dioxid derivative, a carbodimide derivative, a fluorenylidene methane derivative, a distyrylpyrazine derivative, heterocyclic tetracarboxylic anhydride such as a naphthalene perylene derivative, a phthalocyanine derivative, a metal complex or a metallo phthalocyanine of 8-quinolinol derivative, various metal complexes represented by a metal complex having a ligand of benzoxazole or benzothiazole, a polysilane derivative, conductive oligomer such as a poly (N-vinylcarbazole) derivative, an aniline based copolymer, a thiophene oligomer, a poluthiophene derivative, a polyphenylene derivative, a polyphenylene vinylene derivative, a polyfluorene derivative and the like. The host compounds may be used singly or in combination of two or more.
More specific examples include the following:

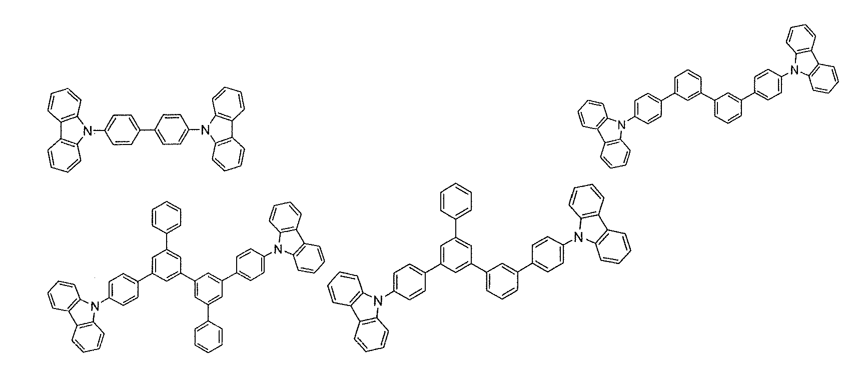

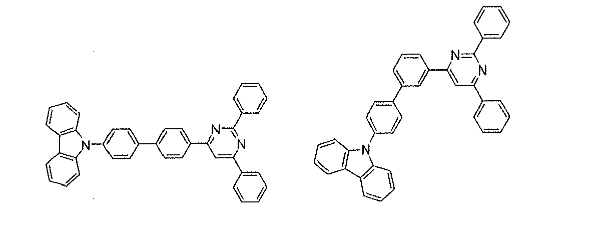

The phosphorescent dopant is a compound capable of emitting light from the triplet exciton. Although it is not restricted as long as it emits light from the triplet exciton, it is preferable that a metal complex comprises at least a metal selected from the group of Ir, Ru, Pd, Pt, Os and Re. A porphyrin metal complex or an orthometalized metal complex is particularly preferable. As the porphyrin metal complex, a porphyrin platinum complex is preferable. The phosphorescent compound may be employed singly or in combination of two or more.
Although there are various ligands to form an orthometalized metal complex, preferred includes 2-phenylpyridine derivatives, 7,8-benzoquinolin derivatives, 2-(2-thienyl) pyridine derivatives, 2-(1-naphthyl) pyridine derivatives, 2-phenylquinokin derivatives and the like. The derivatives may have substituent as appropriate. In particular, the derivatives having a fluorinated compound or a trifluoromethyl group are preferable for a blue hue dopant. In addition, a ligand other than the above ligand such as acetylacetonate and picric acid may be contained as an auxiliary ligand.
The amount of the phosphorescent dopant in the light emitting layer may be selected for the objective as appropriate without particularly restricted, and for example, it may be selected in the range of from 0.1 to 70 % by mass, preferably in the range of from 1 to 30 % by mass. The emission is faint and the advantage is not demonstrated when the amount is less than 0.1 % by mass. The concentration quenching becomes noticeable so that the device performance is deteriorated when the amount is more than 70 % by mass.
Further, the light emitting layer may contain a hole transporting material, a electron transporting material or a polymer binder as appropriate.
Furthermore, the thickness of the light emitting layer is, in general, selected in the range of from 5 to 50 nm, preferably in the range of from 7 to 50 nm and more preferably in the range of from 10 to 50 nm. It is resulted in difficult to form the light emitting layer and to control chromaticity thereof when the thickness is less than 5 nm, and it may be resulted in danger of increasing driving voltage when it is more than 50 nm.

### (5) Hole injecting / transporting layer (Hole transporting zone)

In the present invention, the hole injecting / the hole transporting layer is a layer which assist injection of holes into the light emitting layer and transport the holes to the light emitting zone. The layer exhibits a great mobility of holes and, in general, have an ionization energy as small as 5.5 eV or smaller. For the hole injecting / the hole transporting layer, a material which transports holes to the light emitting layer at a small strength of the electric field is preferable. A material which exhibits, for example, a mobility of holes of at least 10⁻⁴ cm²/V· sec under application of an electric field of from 10⁴ to 10⁶ V/cm is preferable.
When the aromatic amine derivatives are employed for hole transporting zone, they may be used singly or in combination with other material to form a hole injecting / transporting layer.
With regard to the material which may be employed for forming the hole injecting / transporting layer in combination with the aromatic amine derivatives, any material having the foregoing preferable properties is employed without particularly restricted, Any arbitrary material selected from conventional material commonly used as a charge transporting material for the holes in photoconducting materials and well known material employed for the hole injecting / transporting layer in the EL device may be employed.

Further examples include triazole derivatives (refer to U. S. Patent No. 3,112,197, etc.), oxadiazole derivatives (refer to U. S. Patent No. 3,189,447, etc.), imidazole derivatives (refer to Japanese Examined Patent KOKOKU No. Shou 37-16096, etc.), poly arylalkane derivatives (refer to United States Patent Nos. 3,615,402, 3,820,989 and 3,542,544, Japanese Examined Patent KOKOKU Nos. Shou 45-555 and Shou 51-10983, Japanese Unexamined Patent Application Laid-Open Nos. Shou 51-93224, Shou 55-17105, Shou 56-4148, Shou 55-108667, Shou 55-156953, Shou 56-36656, etc.), pyrazoline derivatives and pyrazolone derivatives (refer to U.S Patent Nos. 3,180,729 and 4,278,746, Japanese Unexamined Application Patent Laid-Open Nos. Shou 55-88064, Shou 55-88065, Shou 49-105537, Shou 55-51086, Shou 56-80051, Shou 56-88141, Shou 57-45545, Shou 54-112637, Shou 55-74546, etc.), phenylenediamine derivatives (refer to U.S Patent No. 3,615,404, Japanese Examined Patent KOKOKU Nos. Shou 51-10105, Shou 46-3712 and Shou 47-25336, Japanese Unexamined Patent Application Laid-Open Nos. Shou 54-53435, Shou 54-110536, Shou 54-119925, etc.), arylamine derivatives (refer to U.S Patent Nos. 3,567,450, 3,180,703, 3,240,597, 3,658,520, 4,232,103, 4,175,961 and 4,012,376, Japanese Examined Patent KOKOKU Nos. Shou 49-35702 and Shou 39-27577, Japanese Unexamined Patent Application Laid-Open Nos. Shou 55-144250, Shou 56-119132 and Shou 56-22437, West German Patent No. 1,110,518, etc.), chalcone derivatives which is substituted with amino group (refer to U.S Patent No. 3,526,501, etc.), oxazole derivatives (disclosed in U.S Patent No. 3,257,203, etc.), styryl anthracene derivatives (refer to Japanese Unexamine Patent Application Laid-Open No. Shou 56-46234, etc.), fluorenone derivatives (refer to Japanese Unexamined Patent Application Laid-Open No. Shou 54-110837, etc.), hydrazone derivatives (refer to U.S Patent Nos. 3,717,462, Japanese Unexamined Patent Application Laid-Open Nos. Shou 54-59143, Shou 55-52063, Shou 55-52064, Shou 55-46760, Shou 55-85495, Shou 57-11350, Shou 57-148749, Hei 2-311591, etc.), stilbene derivatives (refer to Japanese Unexamined Patent Application Laid-Open Nos. Shou 61-210363, Shou 61-228451, Shou 61-14642, Shou 61-72255, Shou 62-47646, Shou 62-36674 , Shou 62-10652, Shou 62-30255, Shou 60-93455, Shou 60-94462, Shou 60-174749, Shou 60-175052, etc.), silazane derivatives (U.S Patent No. 4,950,950), polysilane-based copolymers (Japanese Unexamined Patent Application Laid-Open No. Hei 2-204996), aniline-based copolymers (Japanese Unexamined Patent Application Laid-Open No. Hei 2-282263), an electroconductive polymer oligomer (particularly, thiophene oligomer) which is disclosed in Japanese Unexamined Patent Application Laid-Open No Hei 1-211399 and the like.

With regard to the material of the hole injecting / transporting layer, the above materials are also employable, however, porphyrin compounds (published in Japanese Unexamined Patent Application Laid-Open Nos. Shou 63-2956965, etc.), aromatic tertiary amine compounds and styryl amine compounds (refer to U.S Patent No. 4,127,412, Japanese Unexamined Patent Application Laid-Open Nos. Shou 53-27033, Shou 54-58445, Shou 54-149634, Shou 54-64299, Shou 55-79450, Shou 55-144250, Shou 56-119132, Shou 61-295558, Shou 61-98353, Shou 63-295695, etc.) are preferable and the aromatic tertiary amine compounds are particularly preferable.
Further examples include, for example, 4,4'-bis (N-(1-naphthyl) -N-phenylamino) biphenyl (abbreviated as NPD hereunder) having 2 fused aromatic rings in its molecular described in U.S Patent No. 5,061,569, 4,4',4"-tris (N-(3-methylphenyl) -N-phenylamino) triphenyl amine (abbreviated as MTDATA hereunder) made by connecting three triphenyl amine units to form a star burst type and the like.
Further, in addition to the aforementioned asymmetric pyrene derivatives described as a material for the light emitting layer, inorganic compound such as p-type silicon, p-type silicon carbide or the like is employable as the material for the hole injecting layer.

To form the hole injecting / the hole transporting layer, a thin film may be formed from the material for the hole injecting layer or the hole transporting layer, respectively, in accordance with a well known process such as the vacuum vapor deposition process, the spin coating process, the casting process and the LB process. Although the thickness of the hole injecting / the hole transporting layer is not particularly limited, the thickness is usually from 5 nm to 5 *µ*m. The hole injecting / transporting layer may be constructed by a layer comprising at least one of the aforementioned materials or by laminating a hole injecting / transporting layer comprising a different compound other than the aforementioned hole injecting / transporting layer.
In the organic EL device of the present invention, the organic semiconductor layer assists to inject the holes or to inject the electrons into the light emitting layer, and it is preferable for the organic semiconductor layer to have a electric conductivity of 10⁻¹⁰ S/cm or greater. With regard to a material for the organic semiconductor layer, electroconductive oligomer such as an oligomer having thiophene, an oligomer having arylamine disclosed in Japanese Unexamined Patent Application Laid-Open No. 8-193191 and the like, electroconductive dendrimers such as a dendrimer having an arylamine dendrimer are employable.

### (6) Electron injecting / transporting layer

Next, the electron injection / transporting layer in the organic EL device of the present invention is a layer which assists injection of electrons into the light emitting layer and transportation thereof to the light emitting zone, and exhibits a great mobility of electrons. Among the electron injecting layers, an adhesion improving layer is a layer made of a material exhibiting excellent adhesion with the cathode.
Further, it is known that because the emitted light reflects on the electrode (in the above case, on the cathode) in the organic EL device, the light taken out directly through the anode and the light taken out from the electrode (cathode) via reflection interferes each other. In order for utilizing the interference effect effectively, the electron transporting layer is appropriately selected to be several nm to several *µ* m in thickness. However, when the film thickness is thick, a material which exhibits, for example, a mobility of holes of at least 10⁻⁵ cm²/ V · sec under application of an electric field of from 10⁴ to 10⁶ V/cm is preferable for the purpose of evading an elevation of driving electric voltage.
As the material for the electron injecting layer, 8-hydroxyquinoline, metal complexes of derivatives thereof and oxadiazole derivatives are preferable. Examples of the 8-hydroxyquinoline and metal complexes of derivatives thereof include metal chelate of oxinoid compounds including chelate of oxine (in general, 8-quinolinol or 8-hydroxyquinoline). For example, tris(8-quinolinol)aluminum (Alq) can be employed as the electron injecting material.

On the other hand, examples of the oxadiazole derivatives include an electron transfer compound shown as following general formulae: 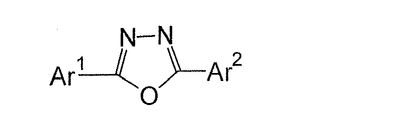 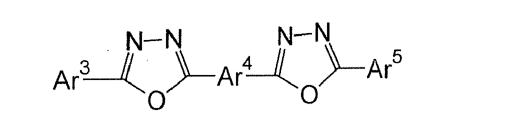 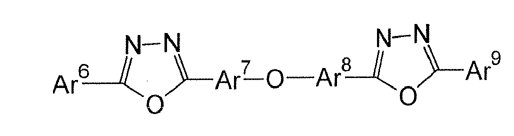 In the general formulae above, Ar¹, Ar², Ar³, Ar⁵, Ar⁶ and Ar⁹ each independently represents a substituted or unsubstituted aryl group respectively, which may be the same with or different from each other; Ar⁴, Ar⁷ and Ar⁸ each independently represents a substituted or unsubstituted arylene group, which may be the same with or different from each other.
Examples of the aryl group include a phenyl group, a biphenyl group, an anthranil group, a perilenyl group and a pyrenyl group. Further, examples of the arylene group include a phenylene group, a naphthylene group, a biphenylene group, an anthranylene group, a perilenylene group, a pyrenylene group and the like. Furthermore, examples of the substituent include an alkyl group having 1 to 10 carbon atoms, an alkoxy group having 1 to 10 carbon atoms or a cyano group and the like. With regard to the electron transfer compounds, the compounds having a thin film forming capability are preferable.

Specific examples of the electron transfer compounds are shown below: 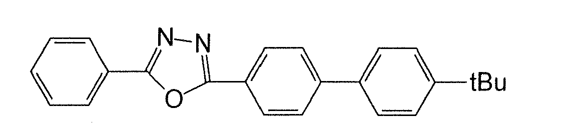 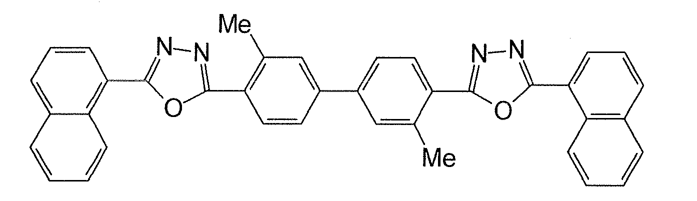 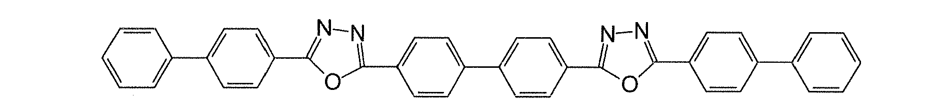 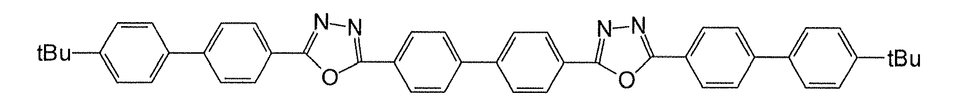 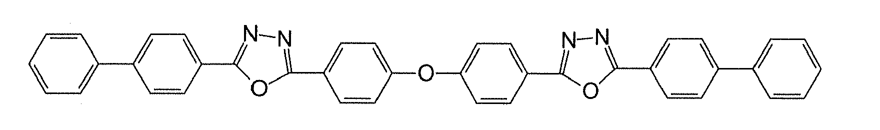 Further, materials shown by following general formulae (A) to (F) are employable for the electron injecting layer and the electron transporting layer.
A heterocyclic derivative having a nitrogen atom represented by a general formula (A) below or a general formula (B) below:

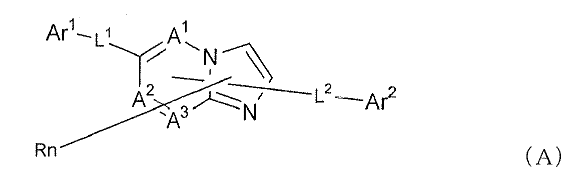

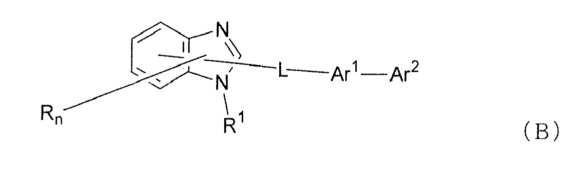

In the general formulae (A) and (B), A¹ to A³ each independently represents a nitrogen atom or a carbon atom;
Ar¹ represents a substituted or unsubstituted aryl group having 6 to 60 ring carbon atoms or a substituted or unsubstituted heteroaryl group having 3 to 60 ring carbon atoms; Ar² represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, a substituted or unsubstituted alkoxy group having 1 to 20 carbon atoms or divalent groups thereof. However, at least one of Ar¹ or Ar² represents a substituted or unsubstituted condensed ring group having 10 to 50 ring carbon atoms or a substituted or unsubstituted monohetero condensed ring group having 3 to 60 ring carbon atoms;
L¹, L² and L each independently represents a single bond, a substituted or unsubstituted arylene group having 6 to 60 ring carbon atoms, a substituted or unsubstituted heteroarylene group having 3 to 60 ring carbon atoms or a substituted or unsubstituted fluorenylene group;
R represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, or a substituted or unsubstituted alkoxy group having 1 to 20 carbon atoms; n represents an integer of 0 to 5; when n is 2 or greater, plural of R may be the same with or different from each other; and adjacent couple of the plural of R may bond to form a carbocyclic aliphatic ring or a carbocyclic aromatic ring.

A heterocyclic derivative having a nitrogen atom represented by a following general formula (C):

HAr-L-Ar¹-Ar² (C)

wherein HAr represents a heterocyclic group having nitrogen atom having 3 to 40 carbon atoms and further may have a substituent; L represents a single bond, a substituted or unsubstituted arylene group having 6 to 60 carbon atoms and further may have a substituent, a substituted or unsubstituted heteroarylene group having 3 to 60 carbon atoms and further may have a substituent or a substituted or unsubstituted fluorenylene group and further may have a substituent; Ar¹ represents a divalent aromatic hydrocarbon group having 6 to 60 carbon atoms and further may have a substituent; and Ar² represents a substituted or unsubstituted aryl group having 6 to 60 carbon atoms, a substituted or unsubstituted heteroaryl group having 3 to 60 carbon atoms and further may have a substituent.

A silacyclopentadiene derivative represented by a following general formula (D):

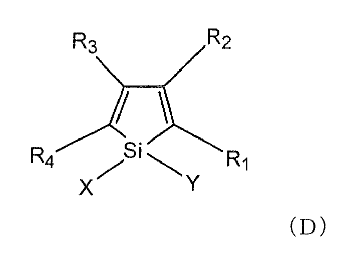

wherein X and Y each independently represents a saturated or unsaturated hydrocarbon group having 1 to 6 carbon atoms, an alkoxy group, an alkenyloxy group, an alkynyl oxy group, a hydroxy group, a substituted or unsubstituted aryl group, a substituted or unsubstituted hetero ring, or a structure forming a saturated or unsaturated ring by bonding X and Y; R₁ to R₄ each independently represents a hydrogen atom, a halogen atom; a substituted or unsubstituted alkyl group having 1 to 6 carbon atoms, an alkoxy group, an aryloxy group, a perfluoroalkyl group, a perfluoro alkoxy group, an amino group, an alkylcarbonyl group, an arylcarbonyl group, an alkoxycarbonyl group, an aryloxycarbonyl group, an azo group, an alkylcarbonyloxy group, an aryloxycarbonyl group, alkoxycarbonyloxy group, aryloxycarbonyloxy group, a sulfinyl group, a sulfonyl group, a sulfanilic group, a silyl group, a carbamoyl group, an aryl group, a heterocyclic group, an alkenyl group, an alkynyl group, a nitro group, a formyl group, a nitroso group, a formyloxy group, an isocyano group, a cyanate group, an isocyanate group, a thiocyanate group, an isothiocyanate group or a cyano group; or in an neighboring case, a structure made by condensing a substituted or unsubstituted ring.

A borane derivative represented by a following general formula (E):

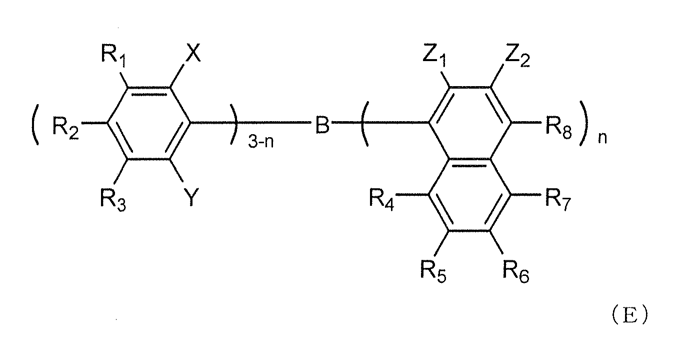

wherein R₁ to R₈ and Z₂ each independently represents a hydrogen atom, a halogen atom, a saturated or unsaturated hydrocarbon group, an aromatic group, a hetero ring group, substituted amino group, a substituted boryl group, an alkoxy group or an aryloxy group; X, Y and Z₁ each independently represents a saturated or unsaturated hydrocarbon group, an aromatic group, a hetero ring group, substituted amino group, an alkoxy group or an aryloxy group; substituent of Z₁ and Z₂ may bonds each other to form a condensed ring; n represents an integer of 1 to 3, and when n is 2 or more, plural of Z₁ may be different from each other. However, a case where n is 1, where X, Y and R₂ are methyl groups, and where R₈ is a hydrogen atom or a substituted boryl group and a case where n is 3 and where Z₁ is a methyl group are excluded.

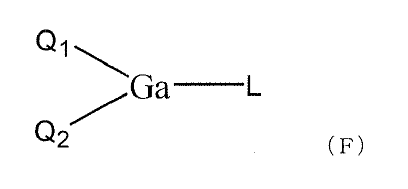

In the general formula (F), Q₁ and Q₂ each independently represents a ligand expressed by a following general formula (G), L represents a halogen atom, a saturated or unsaturated alkyl group, a saturated or unsaturated cycloalkyl group, a saturated or unsaturated aryl group, a saturated or unsaturated heterocyclic group, and -OR¹ (R¹ represents a hydrogen atom, a saturated or unsaturated alkyl group, a saturated or unsaturated cycloalkyl group, a saturated or unsaturated aryl group, a saturated or unsaturated heterocyclic group) or a ligand represented by -O-Ga-Q³(Q⁴); wherein Q³ and Q⁴ are the same as Q₁ and Q₂.

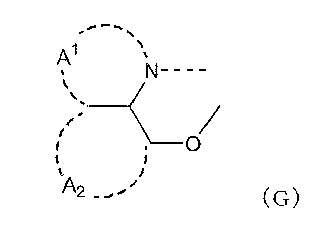

wherein A¹ and A² each represents a condensed 6 member aryl ring structure which may be substituted.

The metal complex is powerfully characterized as n type semiconductor, and its electron injection capability is exciting. In addition, because generation energy in complex formation is small, bonding property between the metal in the formed metal-complex and the ligand becomes strong, and as a result, fluorescence quantum efficiency as the light emitting material also becomes great.
Specific examples of substituent of rings A¹ and A² each forming the ligand of the general formula (G) include halogen atoms such as chlorine atom, bromine atom, iodine atom and fluorine atom; substituted or unsubstituted alkyl group such as methyl group, ethyl group, propyl group, butyl group, sec-butyl group, tert- butyl group, pentyl group, hexyl group, heptyl group, octyl group, stearyl group, trichloromethyl group, and the like; substituted or unsubstituted aryl group such as phenyl group, naphthyl group, 3-methylphenyl group, 3-methoxyphenyl group, 3-fluorophenyl group, 3-trichloromethylphenyl group, 3-trifluoromethylphenyl group, 3-nitrophenyl group and the like; a substituted or unsubstituted alkoxy group such as methoxy group, n-butoxy group, tert-butoxy group, trichloromethoxy group, trifluoroethoxy group, pentafluoropropoxy group, 2,2,3,3-tetrafluoropropoxy group, 1,1,1,3,3,3-hexafluoro-2-propoxy group, 6-(perfluoroethyl)hexyloxy group and the like; a substituted or unsubstituted aryloxy group such as phenoxy group, p-nitrophenoxy group, p-tert-butylphenoxy group, 3-fluorophenoxy group, pentafluorophenyl group, 3-trifluoromethylphenoxy group and the like; a substituted or unsubstituted alkylthio group such as methylthio group, ethylthio group, tert-butylthio group, hexylthio group, octylthio group, trifluoromethylthio group and the like; a substituted or unsubstituted arylthio group such as phenylthio group, p-nitrophenylthio group, ptert-butylphenylthio group, 3-fluorophenylthio group, pentafluorophenylthio group, 3- trifluoromethylphenylthio group and the like; a mono- or di-substituted amino group such as cyano group, nitro group, amino group, methylamino group, diethylamino group, ethylamino group, diethylamino group, dipropylamino group, dibutyl amino group, diphenylamino group and the like; acylamino-group such as bis(acetoxymethyl)amino group, bis(acetoxyethyl)amino group, bis(acetoxypropyl)amino group, bis(acetoxybutyl) amino group and the like ; carbamoyl group such as hydroxy group, siloxy group, acyl group, methylcarbamoyl group, dimethylcarbamoyl group, ethylcarbamoyl group, diethylcarbamoyl group, a propylcarbamoyl goup, butyl carbamoyl group, a phenylcarbamoyl group and the like; cycloalkyl group such as carboxylic acid group, sulfonic acid group, imido group, cyclopentane group, cyclohexyl group, etc.; aryl group such as phenyl group, naphthyl group, biphenyl group, anthranil group, phenanthryl group, fluorenyl group, pyrenyl group and the like; heterocyclic group such as pyridinyl group, pyrazinyl group, pyrimidinyl group, pyridazinyl group, triazinyl group, indolinyl group, quinolinyl group, acridinyl group, pyrrolidinyl group, dioxanyl group, piperidinyl group, morpholidinyl group, piperazinyl group, triazinyl group, carbazolyl group, furanyl group, thiophenyl group, oxazolyl group, an oxadiazolyl group, a benzoxazolyl group, a thiazolyl group, a thiadiazolyl group, benzothiazolyl group, triazolyl group, imidazolyl group, benzimidazolyl group, pranyl group and the like. Further, aforementioned substituents may bond each other to form further a 6 member aryl ring or a hetero ring.

As an organic EL device of the present invention, it is preferable that a reductive dopant is added in either the electron transporting zone or an interfacial zone between the cathode and the organic layer. The reductive dopant used in the present invention is defined as a substance which reduces the electron transporting compound. Therefore, various compounds may be employed if they have a certain level of reduction capability. Examples of the preferable reductive dopant include at least one compound selected from the group comprising alkali metals, alkaline earth metals, rare earth metals, alkali metal oxides, alkali metal halides, alkaline earth metal oxides, alkaline earth metal halides, rare earth metal oxides, rare earth metal halides, organic complexes of alkali metals, organic complexes of alkaline earth metals and organic complexes of rare earth metals.
Examples of the more preferable reductive dopant include at least one alkali metal selected from a group consisting of Na (the work function: 2.36 eV), K (the work function: 2.28 eV), Rb (the work function: 2.16 eV) and Cs (the work function: 1.95 eV) or at least one alkaline earth metals selected from a group consisting of Ca (the work function: 2.9eV), Sr (the work function: 2.0 to 2.5 eV) and Ba (the work function: 2.52eV); whose work function of 2.9 eV or smaller is particularly preferable. Among those, more preferable reductive dopant include at least one kind or more alkali metal selected from the group consisting of K, Rb and Cs, the latter Rb or Cs being farther more preferable and the last Cs being the most preferable. Those alkali metals have particularly high reducing capability, and only an addition of relatively small amount of them into an electron injection zone enables to achieve both improvement of luminance and lifetime extension of the organic EL device. Further, with regard to the reductive dopant with work function of 2.9 eV or smaller, a combination of two or more kinds of the alkali metal is also preferable, and particularly, combinations containing Cs, for example, combinations of Cs and Na, Cs and K, Cs and Rb, or Cs and Na and K are preferable. Containing Cs in combination enables to reveal reducing capability effectively, and the addition into the electron injection zone expects both improvement of luminance and lifetime extension of the organic EL device.

In the organic EL device of the present invention, an electron injecting layer formed with an insulating material or a semiconductor may be further sandwiched between the cathode and the organic thin film layer. The electron injecting layer effectively prevents leak in the electric current and improves the electron injecting capability. It is preferable that at least one metal compound selected from the group consisting of alkali metal chalcogenides, alkaline earth metal chalcogenides, alkali metal halides and alkaline earth metal halides is used as the insulating material. It is preferable that the electron injecting layer is constituted with the above alkali metal chalcogenide since the electron injecting capability can be improved. Preferable examples of the alkali metal chalcogenide include Li2O, LiO, Na₂S, Na₂Se and NaO. Preferable examples of the alkaline earth metal chalcogenide include CaO, BaO, SrO, BeO, BaS and CaSe. Preferable examples of the alkali metal halide include LiF, NaF, KF, LiCl, KCl and NaCl. Preferable examples of the alkaline earth metal halide include fluorides such as CaF₂, BaF₂, SrF₂, MgF₂ and BeF₂ and halides other than the fluorides.
Examples of the semiconductor constituting the electron transporting layer include oxides, nitrides and oxide nitrides containing at least one element selected from Ba, Ca, Sr, Yb, Al, Ga, In, Li, Na, Cd, Mg, Si, Ta, Sb and Zn, which are used singly or in combination of two or more. It is preferable that the inorganic compound constituting the electron transporting layer is in the form of a fine crystalline or amorphous insulating thin film. When the electron transporting layer is constituted with the above insulating thin film, a more uniform thin film can be formed and defective pixels such as dark spots can be decreased. Examples of the inorganic compound include the alkali metal chalcogenides, the alkaline earth metal chalcogenides, the alkali metal halides and the alkaline earth metal halides which are described above.

### (7) Cathode

As the cathode for the organic EL device of the present invention, an electrode substance such as metal, alloy, electroconductive compound and those mixture having a small work function (4 eV or smaller) is employed. Examples of the electrode substance include potassium, sodium- potassium alloy, magnesium, lithium, magnesium-silver alloy, aluminum / aluminum oxide, aluminum-lithium alloy, indium, rare earth metal and the like. The cathode can be prepared by forming a thin film of the electrode material described above in accordance with a process such as the vapor deposition process and the sputtering process.
When the light emitted from the light emitting layer is observed through the anode, it is preferable that the anode has a transmittance of the emitted light greater than 10 %. It is also preferable that the sheet resistivity of the anode is several hundred Ω /□ or smaller. The thickness of the anode is, in general, selected in the range of from 10 nm to 1 µm and preferably in the range of from 50 to 200 nm.

### (8) Insulating layer

An organic EL device tends to form defects in pixels due to leak and short circuit since an electric field is applied to ultra-thin films. To prevent the formation of the defects, a layer of an insulating thin film may be inserted between the pair of electrodes.
Examples of the material employed for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide and vanadium oxide. Mixtures and laminates of the above compounds may also be employed.

### (9) Process of fabricating an organic EL device

To fabricate an organic EL device of the present invention, for example, an anode, a light emitting layer and, where necessary, a hole injecting / transporting layer, and where necessary, an electron injecting / transporting layer may be formed in accordance with the aforementioned process using the aforementioned materials, and a cathode is formed in the last step. An organic EL device may be produced by forming the aforementioned layers in the order reverse to that described above, i.e., a cathode being formed in the first step and an anode in the last step.
An embodiment of the process for fabricating an organic EL device having a construction in which an anode, a hole injecting layer, a light emitting layer, an electron injecting layer and a cathode are disposed sequentially on a light-transmitting substrate will be described in the following.
On a suitable substrate which transmits light, a thin film made of a material for the anode is formed in accordance with the vapor deposition process or the sputtering process so that the thickness of the formed thin film is 1 *µ*m or smaller and preferably in the range of 10 to 200 nm. The formed thin film is employed as the anode. Then, a hole injecting layer is formed on the anode. The hole injecting layer can be formed in accordance with the vacuum vapor deposition process, the spin coating process, the casting process or the LB process, as described above. The vacuum vapor deposition process is preferable since a uniform film can be easily obtained and the possibility of formation of pin holes is small. When the hole injecting layer is formed in accordance with the vacuum vapor deposition process, in general, it is preferable that the conditions in general are suitably selected in the following ranges: temperature of the deposition source: 50 to 450°C; vacuum level: 10⁻⁷ to 10⁻³ torr; deposition rate: 0.01 to 50 nm/second; temperature of the substrate: -50 to 300°C; and film thickness: 5 nm to 5 µm; although the conditions of the vacuum vapor deposition are different depending on the employed compound (the material for the hole injecting layer) and the crystal structure and the recombination structure of the hole injecting layer to be formed.

Subsequently, the formation of the light emitting layer on the hole-injecting layer can be made by forming the desired light emitting material into a thin film in accordance with the vacuum vapor deposition process, the sputtering process, the spin coating process or the casting process. The vacuum vapor deposition process is preferable because a uniform film can be easily obtained and the possibility of formation of pinholes is small. When the light emitting layer is formed in accordance with the vacuum vapor deposition process, in general, the conditions of the vacuum vapor deposition process can be selected in the same ranges as those described for the vacuum vapor deposition of the hole injecting layer although the conditions are different depending on the used compound.
Next, the electron injecting layer is formed on the light emitting layer formed above. Similarly to the hole injecting layer and the light emitting layer, it is preferable that the electron injecting layer is formed in accordance with the vacuum vapor deposition process since a uniform film should be obtained. The conditions of the vacuum vapor deposition can be selected in the same ranges as those for the hole injecting layer and the light emitting layer.
Although the aromatic amine derivatives depend on that it is contained in a light emitting layer or a hole transporting layer, it may be vapor deposited together with other materials. In addition, when the spin coating process is employed, it may be contained therein by blending it with other materials.
An organic EL device is produced by laminating a cathode as the last step. The anode is made of a metal and can be formed in accordance with the vacuum vapor deposition process or the sputtering process. It is preferable that the vacuum vapor deposition process is employed in order to prevent the lower organic layers from damages during the formation of the film.
In the above production of the organic EL device, it is preferable that the above layers from the anode to the cathode are formed successively while the production system is kept in a vacuum after being evacuated.

The process for forming the layers in the organic EL device of the present invention is not particularly limited. A conventional process such as the vacuum vapor deposition process and the spin coating process can be used. The organic thin film layer comprising the compound represented by the foregoing general formula (1) used in the organic EL device of the present invention can be formed in accordance with the vacuum vapor deposition process, the molecular beam epitaxy process (the MBE process) or, using a solution prepared by dissolving the compound into a solvent, in accordance with a conventional coating process such as the dipping process, the spin coating process, the casting process, the bar coating process and the roller coating process.
The thickness of each layer in the organic thin film layer in the organic EL device of the present invention is not particularly limited. In general, an excessively thin layer tends to have defects such as pin holes, and an excessively thick layer requires a high applied voltage results in decreasing the efficiency. Therefore, a thickness within the range of several nanometers to 1 µm is preferable.
The organic EL device which can be produced as described above emits light when a direct voltage of 5 to 40 V is applied in the condition that the anode is connected to a positive electrode (+) and the cathode is connected to a negative electrode (-). When the connection is reversed, no electric current is observed and no light is emitted at all. When an alternating voltage is applied to the organic EL device, the uniform light emission is observed only in the condition that the polarity of the anode is positive and the polarity of the cathode is negative. When an alternating voltage is applied to the organic EL device, any type of wave shape can be employed.

### Example

This invention will be described in further detail with reference to the examples, which do not limit the scope of this invention.

### Synthesis Instance 1 (Synthesis of Intermediate 1)

Into a three neck flask of 200 ml, 20 g of 4-bromobiphenyl (manufactured by Tokyo Kasei Kogyo Co., Ltd.), 8.64 g of sodium-t-butoxide (manufactured by Wako Pure Chemical Industries, Ltd.) and 84 mg of palladium acetate (manufactured by Wako Pure Chemical Industries, Ltd.) were placed, followed by placing a stirrer therein. Subsequently, the two necks at both sides of the flask were covered by rubber cups, and then a reflux coiled-tube was placed in the remaining neck. A three-way stopcock was inserted in the top of the tube and a balloon filed with argon gas was set at one way of the stopcock, followed by three time argon replacement of the inside of the flask by using a vacuum pump.
Then, 120 ml of dehydrated toluene (manufactured by Hiroshima-Wako Co., Ltd.), 4.08 ml of benzylamine (manufactured by Tokyo Kasei Kogyo Co., Ltd.), 338 micron litter (manufactured by Aldrich Corporation; 2.22 mol/l of toluene solution) were put into the flask though a rubber septum by using a syringe, followed by stirring for 5 minutes at room temperature.
Subsequently, the flask was put in a oil bath and heated gradually up to 120 °C with stirring. After 7 hours passed, the flask was set aside from the oil bath so as to stop the reaction, and it was left for 12 hours.
The reaction solution was placed into a separating funnel, and the precipitation was dissolved by adding 600 ml of dichloromethane. It was washed by 120 ml of saturated salt water and the organic layer was dried with the use of potassium carbonate anhydride. The solvent of the organic layer obtained by filtrating potassium carbonate was removed though distillation. Then 400 ml of toluene and 80 ml of ethanol were added in the residue, and a drying tube was set thereto, followed by heating up to 80 °C so as to dissolve the residue completely. Then, while it was left for 12 hours, it was gradually cooled down to room temperature for recrystallization thereof.
By separating the precipitated crystal through filtration and drying it at 60 °C under vacuum, 13.5 g of N,N-di-(4-biphenylyl)-benzylamine was obtained.
Into a neck flask with 300ml, 1.35 g of N,N-di-(4-biphenylyl)-benzylamine, 135 g of palladium-active carbon (manufactured by Hiroshima-Wako Co., Ltd.; containing 10 % by weight of palladium) were placed, and then were dissolved by adding 100 ml of chloroform and 20 ml of ethanol.
Subsequently, after placing a stirrer therein, three-way stopcock, of which one way was set with a balloon filed with 2 litter of hydrogen gas, was set to the flask, followed by 10 time hydrogen gas displacement of the inside of the flask by using a vacuum pump. The balloon was refilled to be 2 litters with hydrogen gas, followed by stirring the solution intensively at room temperature. After stirring for 30 hours, 100 m of dichloromethane was added therein, and the catalyst was separated by filtration.
Subsequently the obtained solution was put in a separating funnel, followed by washing with 50 ml of a saturated sodium carbonate aqueous solution. Then the organic layer was separated and dried with the use of potassium carbonate anhydride. The solvent obtained by filtrating potassium carbonate was removed though distillation. Then 50ml of toluene was added in the residue for recrystallization thereof. By separating the precipitated crystal through filtration and vacuum drying it at 50 °C, 0.99 g of di-4-biphenylylamine (Intermediate 1, which will be illustrated later) was obtained.

### Synthesis Instance 2 (Synthesis of Intermediate 2)

Under the argon gas current, 10 g of di-4-biphenylylamine (manufactured by Tokyo Kasei Kogyo Co.,Ltd.), 9.7 g of 4,4'-dibromobiphenyl (manufactured by Tokyo Kasei Co.,Ltd.), 3 g of sodium-t-butoxide (manufactured by Hiroshima-Wako Co., Ltd.), 0.5 g of bis(triphenylphosphine) palladium (II) chloride (manufactured by Tokyo Kasei Kogyo Co., Ltd.) and 500 ml of xylene were placed, and then they were reacted at 130 °C for 24 hours.
After cooled it down, 1000 ml of water was added, followed by filtration of the mixture by using Celite. Then the solution obtained by the filtration was extracted by toluene, followed by drying it with the use of potassium carbonate anhydride. After concentrating it under reduced pressure, the crude product was refined by column refining and the obtained was recrystallized by using toluene. It was separated by filtration and dried, then 4.6 g of 4'-bromo-N,N-dibiphenyl-4-amino-1,1'-biphenyl (Intermediate 2, which will be illustrated later) was obtained.

### Synthesis Instance 3 (Synthesis of Intermediate 3)

Under the argon gas current, 6.8 g of N-phenyl-1-naphthylamine (manufactured by Tokyo Kasei Kogyo Co., Ltd.), 9.7 g of 4,4'-dibromobiphenyl (manufactured by Tokyo Kasei Co.,Ltd.), 3 g of sodium-t-butoxide (manufactured by Hiroshima-Wako Co., Ltd..), 0.5 g of triphenylphosphine palladium (II) chloride (manufactured by Tokyo Kasei Kogyo Co.,Ltd.) and 500 ml of xylene were placed, and then they were reacted at 130 °C for 24 hours.
After cooled it down, 1000 ml of water was added, followed by filtration of the mixture by using Celite. Then the solution obtained by the filtration was extracted by toluene, followed by drying it with the use of magnesium sulfate anhydride. After concentrating it under reduced pressure, the crude product was refined by column refining and the obtained was recrystallized by using toluene. It was separated by filtration and dried, then 4.1 g of 4'-bromo-N-phenyl-N-1-naphthyl-4-amino-1,1'-biphenyl (Intermediate 3, which will be illustrated later) was obtained.

### Synthesis Instance 4 (Synthesis of Intermediate 4 and Intermediate 5)

Into a three neck flask, 250 g of m-terphenyl (manufactured by Aldrich Corporation), 50 g of hydroiodic acid dihydrate, 75 g of iodine, 750ml of acetic acid and 25ml of concentrated sulfuric acid were placed and then they were reacted at 70 °C for 3 hours. After the reaction, 5 litter of methanol was poured therein, followed by stirring for one hour. After separating it with filtration, the crystal was refined by column chromatography and the obtained was recrystallized by using acetonitrile, then 64 g of the following 3'-phenyl-4-bromobihenyl (Intermediate 4, which will be illustrated later) and 17 g of the following 3-phenyl-5-bromobiphenyl (Intermediate 5, which will be illustrated later) were obtained.

### Synthesis Instance 5 (Synthesis of Intermediate 6)

Under the argon gas atmosphere, into a three neck flask of 1000 ml, 50 g of 2-bromofluorene (manufactured by Tokyo Kasei Kogyo Co., Ltd.), 100 ml of dimethysulfoxide (DMSO), 0.95 g of benzyltriethylammonium chloride (manufactured by Hiroshima-Wako Co., Ltd.) and 65 g of sodium hydroxide aqueous solution (50 % by weight) were placed.
The flask was put in a water bath and 44 g of 1,5-dibromopentane was added therein with stirring. After the reaction for 5 hours, 1000 ml of water added therein, extraction by 500 ml of toluene was carried out. The organic layer was dried with the use of magnesium sulfate, followed by removing the solvent by distillation, and 56 g of Intermediate 6, which will be illustrated later, was obtained as oil.

### Synthesis Instance 6 (Synthesis of Intermediate 7)

It was carried out in a similar manner as Synthesis Instance 5 except that 47 g of 1,6-dibromohexane (manufactured by Hiroshima-Wako Co., Ltd.) was used in place of 1,5-dibromopentane and as a result, 49 g of Intermediate 7, which will be illustrated later, was obtained as oil.

### Synthesis Instance 7 (Synthesis of Intermediate 8)

Under argon gas atmospheric current, 8 g of 9-bromophenanthrene (manufactured by Tokyo Kasei Kogyo Co., Ltd.), 6.0 g of sodium-t-butoxide (Hiroshima-Wako Co., Ltd..), 1 g of triphenylphosphine palladium (II) chloride (manufactured by Tokyo Kasei Kogyo Co.,Ltd.) and 900 ml of xylene were placed, and then they were reacted at 130 °C for 24 hours.
After cooled it down, 1500ml of water was added therein, followed by filtration of the mixture by using Celite. Then the solution obtained by the filtration was extracted by toluene, followed by drying it with the use of magnesium sulfate anhydride. After concentrating it under reduced pressure, the crude product was refined by column refining and the obtained was recrystallized by using toluene. It was separated by filtration and dried, and then 6.2 g of pale yellow powder as the following Intermediate 8, which will be illustrated later, was obtained.

### Synthesis Instance 8 (Synthesis of Intermediate 9)

It was carried out in a similar manner as Synthesis Instance 7 except that 9.6 g of the Intermediate 4 was used in place of 8 g of 9-dibromophenanthrene and as a result, 7.4 g of Intermediate 9, which will be illustrated later, was obtained as pale yellow powder.

### Synthesis Instance 9 (Synthesis of Intermediate 10)

It was carried out in a similar manner as Synthesis Instance 7 except that 9.6 g of the Intermediate 5 was used in place of 8 g of 9-dibromophenanthrene and as a result, 6.6 g of Intermediate 10, which will be illustrated later, was obtained as pale yellow powder.

### Synthesis Instance 10 (Synthesis of Intermediate 11)

It was carried out in a similar manner as Synthesis Instance 7 except that 9.6 g of the Intermediate 6 was used in place of 8 g of 9-dibromophenanthrene and as a result, 6.4 g of Intermediate 11, which will be illustrated later, was obtained as pale yellow powder.

### Synthesis Instance 11 (Synthesis of Intermediate 12)

It was carried out in a similar manner as Synthesis Instance 7 except that the Intermediate 7 was used in place of 8 g of 9-dibromophenanthrene 8.2 g of Intermediate 12, which will be illustrated later, was obtained as pale yellow powder.

### Synthesis Instance 12 (Synthesis of Intermediate 13)

Under argon gas current, into a three neck flask with 200 ml, 5.7 g of benzamide (manufactured by Tokyo Kasei Kogyo Co., Ltd.), 10 g of 4-bromobiphenyl (manufactured by Tokyo Kasei Kogyo Co.,Ltd.), 0.82 g of copper (I) iodide (manufactured by Wako Pure Chemical Industries, Ltd.), 0.76 g of N,N'-dimethyldiamine (manufactured by Aldrich Corporation), 11.8 g of potassium carbonate (manufactured by Wako Pure Chemical Industries) and 60 ml of xylene were placed, and then they were reacted at 130 °C for 36 hours.
After cooled it down, it was filtrated and washed by toluene. Further, it was washed by water and methanol, and dried, and then 10.5 g of the following Intermediate 13, which will be illustrated later, was obtained as pale yellow powder.

### Synthesis Instance 13 (Synthesis of Intermediate 14)

Under argon gas current, into a three neck flask with 300 ml, 16.4 g of Intermediate 13, 25.6 g of Intermediate 4, 1.14 g of copper (I) iodide (manufactured by Wako Pure Chemical Industries, Ltd.), 1.06 g of N,N'-dimethyldiamine (manufactured by Aldrich Corporation), 20.0g of potassium carbonate (manufactured by Wako Pure Chemical Industries, Ltd.) and 100 ml of xylene were placed, and then they were reacted at 130 °C for 36 hours. After cooled it down, it was filtrated and washed by toluene. Further, it was washed by water and methanol, and dried, and then 14.0 g of the pale yellow powder was obtained.
Into a three neck flask of 300 ml, 14.0g of the above powder, 15.1 g of potassium hydroxide (manufactured by Wako Pure Chemical Industries, Ltd.), 13 ml of ion exchanged water, 17ml of xylene (manufactured by Wako Pure Chemical Industries, Ltd.) and 9 ml of ethanol (manufactured by Wako Pure chemical Industries, Ltd.) were placed and refluxed for 36 hours. After the reaction was completed, it was extracted by toluene, followed by drying with use of the magnesium sulfate. It was concentrated under reduced pressure, and the obtained crude product was refined with column refining. It was recrystallized with toluene and the crystal was separated by filtration and dried, then 9.3 g of Intermediate 14, which will be illustrated later, was obtained as white powder.

### Synthesis Instance 14 (Synthesis of Intermediate 15)

Under argon gas current, into a three neck flask with 300 ml, 11.1 g of 1-acetamidonaphtharene (manufactured by Tokyo Kasei Kogyo Co.,Ltd.), 25.6 g of Intermediate 4, 1.14 g of copper (I) iodide (manufactured by Wako Pure Chemical Industries, Ltd.), 1.06 g of N,N'-dimethyldiamine (manufactured by Aldrich Corporation), 20.0 g of potassium carbonate (manufactured by Wako Pure Chemical Industries, Ltd.) and 100 ml of xylene were placed, and then they were reacted at 130 °C for 36 hours. After cooled it down, it was filtrated and washed by toluene.
Further, it was washed by water and methanol, and dried, then 15 g of the pale yellow powder was obtained.
Into a three neck flask of 300 ml, 15.0g of the above powder, 17.6 g of potassium hydroxide (manufactured by Wako Pure Chemical Industries, Ltd.), 15 ml of ion exchanged water, 20 ml of xylene (manufactured by Wako Pure Chemical Industries, Ltd.) and 10ml of ethanol (manufactured by Wako Pure chemical Industries, Ltd.) were placed and refluxed for 36 hours. After the reaction was completed, it was extracted by toluene, followed by drying with use of the magnesium sulfate. It was concentrated under reduced pressure, and the obtained crude product was refined with column refining. Re-crystallization with toluene was carried out and the crystal was separated by filtration and dried, then 11.2 g of the following Intermediate 15, which will be illustrated later, was obtained as white powder.

### Synthesis Instance 15 (Synthesis of Intermediate 16)

It was carried out in a similar manner as Synthesis Instance 13 except that 25.6 g of the Intermediate 5 was used in place of 25.6 g of the Intermediate 4 and as a result, 10.3 g of Intermediate 16, which will be illustrated later, was obtained as white powder.

### Synthesis Instance 16 (Synthesis of Intermediate 17)

It was carried out in a similar manner as Synthesis Instance 14 except that 25.6 g of the Intermediate 5 was used in place of 25.6 g of the Intermediate 4 and as a result, 12.1 g of Intermediate 17, which will be illustrated later, was obtained as white powder.

### Synthesis Instance 17 (Synthesis of Intermediate 18)

Under argon gas current, into a three neck flask with 200ml, 7.2 g of benzamide (manufactured by Tokyo Kasei Kogyo Co.,Ltd.), 40.8 g of the Intermediate 5, 2.3 g of copper (I) iodide (manufactured by Wako Pure Chemical Industries, Ltd.), 2.1 g of N,N'-dimethyldiamine (manufactured by Aldrich Corporation), 33.1 g of potassium carbonate (manufactured by Wako Pure Chemical Industries, Ltd.) and 100 ml of xylene were placed, and then they were reacted at 130 °C for 36 hours. After cooled down, it was washed by toluene. Further, it was washed by water and methanol, and dried, and then 27.0 g of the pale yellow powder was obtained.
Into a three neck flask of 300 ml, 27.0g of the above powder, 19.0 g of potassium hydroxide (manufactured by Wako Pure Chemical Industries, Ltd.), 17 ml of ion exchanged water, 25ml of xylene (manufactured by Wako Pure Chemical Industries, Ltd.) and 12 ml of ethanol (manufactured by Wako Pure chemical Industries, Ltd.) were placed and refluxed for 36 hours. After the reaction was completed, it was extracted by using toluene, and then dried with the use of magnesium sulfate. It was concentrated under reduced pressure, and the obtained crude product was refined with column refining. It was recrystallized with toluene and the crystal was separated by filtration and dried, then 18.1 g of Intermediate 18, which will be illustrated later, was obtained as white powder.

### Synthesis Instance 18 (Synthesis of Intermediate 19)

It was carried out in a similar manner as Synthesis Instance 13 except that 19.7 g of the Intermediate 6 was used in place of 25.6 g of the Intermediate 4 and as a result, 10.5 g of Intermediate 19, which will be illustrated later, was obtained as white powder.

### Synthesis Example 19 (Synthesis of Intermediate 20)

It was carried out in a similar manner as Synthesis Instance 13 except that 20.6 g of the Intermediate 7 was used in place of 25.6 g of the Intermediate 4 and as a result, 11.5 g of the following Intermediate 20, which will be illustrated later was obtained as white powder.

### Synthesis Instance 20 (Synthesis of Intermediate 21)

Under argon gas current, into a three neck flask with 500ml, 31 g of Intermediate 4, 80ml of dehydrated toluene and 80ml of dehydrated ether were placed and it was cooled down to -30 °C.76 ml of n-butyl lithium (n-BuLi, 1.6M hexane solution) was added therein and reacted for 1 hour. After cooling it down to -70 °C, 70 ml of Boric Acid Triisopropyl Ester (manufactured by Tokyo Kasei Kogyo Co., Ltd.) was added therein, and then heated gradually up, followed by stirring for 1 hour at room temperature. After adding 80 ml of 10% hydrochloric acid solution and it was stirred. It was extracted by ethylacetate and the organic layer was washed by water.
It was dried with the use of sodium sulfate anhydride, followed by removing the solvent by distillation. The residue was washed by hexane and the white powder was obtained.
Under argon gas current, into a three neck flask with 500ml, 20 g of the above white powder, 18.6 g of 4-iodebromobiphenyl, 0.8 g of tetrakis triphenylphosphine palladium (Pd(PPh₃)₄), 20.9 g of sodium carbonate, 160 ml of 1,2-dimethoxyethane and 100 ml of H₂O were placed and it was refluxed for 8 hours. It was extracted by toluene and the obtained organic layer was washed by water. The organic layer was dried with the use of sodium sulfate anhydride and the solvent was removed from it by distillation. The recrystallization was carried out by toluene/hexane, and then 15 g of the following Intermediate 21, which will be illustrated later was obtained as white powder.

### Synthesis Instance 21 (Synthesis of Intermediate 22)

It was carried out in a similar manner as Synthesis Instance 13 except that 27.5 g of the Intermediate 21 was used in place of 25.6 g of the Intermediate 4 and as a result, 13 g of Intermediate 21, which will be illustrated later, was obtained as white powder.

### Synthesis Instance 22 (Synthesis of Intermediate 23)

Under argon gas current, into a three neck flask with 300 ml, 20 g of Intermediate 16, 15.6 g of 4-iodebromobiphenyl(manufactured by Wako Pure Chemical Industries, Ltd.), 1.9 g of copper (I) iodide (manufactured by Wako Pure Chemical Industries, Ltd.), 2.0 g of N,N'-dimethyldiamine (manufactured by Aldrich Corporation), 8.6 g of sodium-t-butoxide (manufactured by Tokyo Kasei Kogyo Co., Ltd.) and 100 ml of dehydrated toluene were placed, and then they were reacted at 110 °C for 8 hours. After the reaction was completed, it was extracted by using toluene, and then the extract was dried with use of magnesium sulfate. It was concentrated under reduced pressure, and the obtained crude product was refined with column refining. Re-crystallization with toluene was carried out and the crystal was separated by filtration and dried, then 22.1 g of white powder was obtained.
Under argon gas current, into a three neck flask with 300ml, 22.1 g of the above powder and 100 ml of dehydrated xylene were placed and it was cooled down to -30 °C. 30 ml of n-butyl lithium (n-BuLi, 1.6M hexane solution) was added therein and reacted for 1 hour. After cooling it down to -70 °C, 70 ml of Boric Acid Triisopropyl Ester (manufactured by Tokyo Kasei Kogyo Co., Ltd.), and then heated gradually up, followed by stirring for 1 hour at room temperature. After adding 32 ml of 10% hydrochloric acid solution and it was stirred. It was extracted by using ethylacetate and the organic layer was washed by water. It was dried with the use of sodium sulfate anhydride, followed by removing the solvent by distillation. The residue was washed by hexane and 12.5 g of Intermediate 23, which will be illustrated later, was obtained as white powder.

### Synthesis Instance 23 (Synthesis of Intermediate 24)

It was carried out in a similar manner as Synthesis Instance 22 except that 18.6 g of Intermediate 15 was used in place of 20 g of Intermediate 16 and as a result, 10.6 g of Intermediate 24, which will be illustrated later, was obtained as white powder.

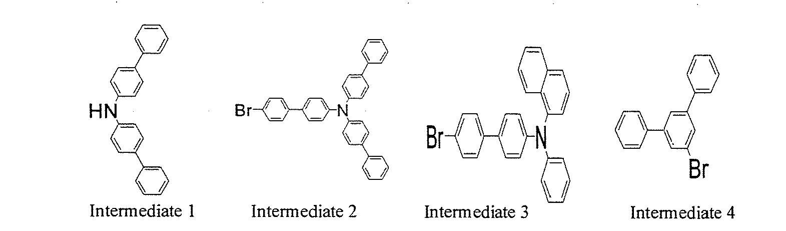

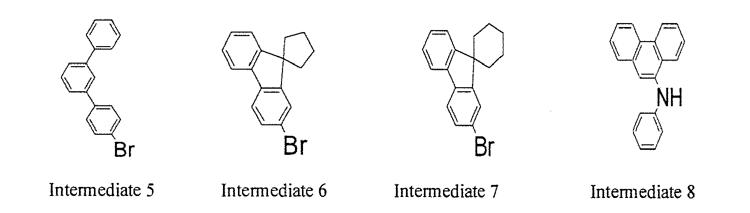

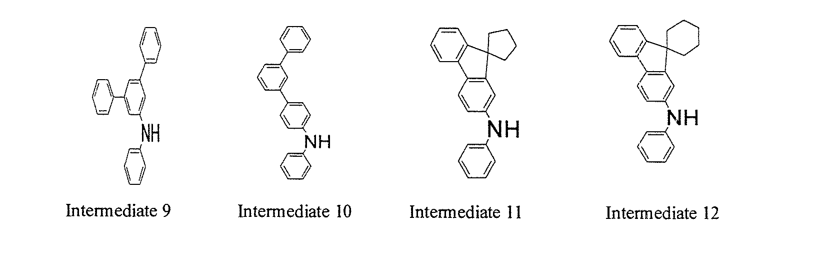

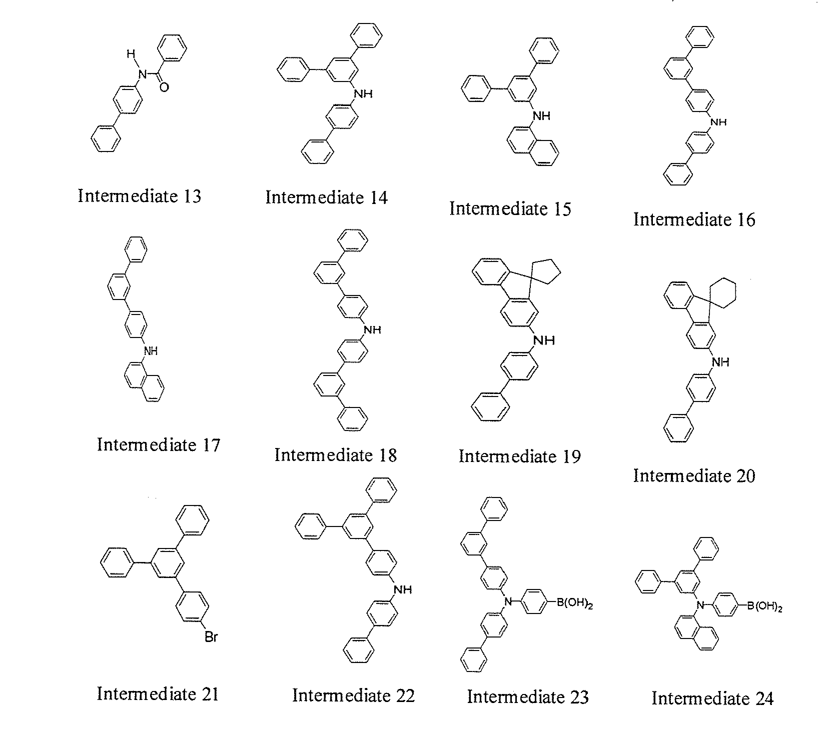

### Synthesis Example 1 (Synthesis of Compound H1)

Under argon gas current, 3.2 g of the Intermediate 8, 5.5 g of Intermediate 2, 2 g of sodium-t-butoxide (manufactured by Hiroshima Wako Co., Ltd.), 0.33 g of bis(triphenylphosphine) palladium (II) chloride (manufactured by Tokyo Kasei Kogyo Co.,Ltd.) and 300 ml of xylene were placed, and then they were reacted at 130 °C for 24 hours.
After cooled it down, 500 ml of water was washed, followed by filtration of the mixture by using Celite. Then the solution obtained by the filtration was extracted by toluene, followed by drying it with the use of potassium carbonate anhydride. After concentrating it under reduced pressure, the crude product was refined by column refining and the obtained was recrystallized by using toluene. It was separated by filtration and dried, and then 3.7 g of the pale yellow powder was obtained. By Field Desorption Mass Spectrometry (FD=MS) analysis, the main peak of m / z = 741 in the case of C₅₆H₄₀N₂ = 741 was obtained, therefore it was identified that the obtained was the foregoing Compound H1.

### Synthesis Example 2 (Synthesis of Compound H5)

It was carried out in a similar manner as Synthesis Example 1 except that 4.5 g of the Intermediate 3 was used in place of 5.5 g of the Intermediate 2 and as a result, 3.9 g of the pale yellow powder was obtained. By FD-MS analysis, the main peak of m / z = 639 in the case of C₄₈H₃₄N₂ = 639 was recognized, therefore it was identified that the obtained was the foregoing Compound H5.

### Synthesis Example 3 (Synthesis of Compound H25)

It was carried out in a similar manner as Synthesis Example 1 except that 3.9 g of the Intermediate 9 was used in place of 3.2 g of the Intermediate 8 and as a result, 4.1 g of the pale yellow powder was obtained. By FD-MS analysis, the main peak of m / z = 793 in the case of C₆₀H₄₄N₂ = 793 was recognized, therefore it was identified that the obtained was the foregoing Compound H25.

### Synthesis Example 4 (Synthesis of Compound H29)

It was carried out in a similar manner as Synthesis Example 1 except that 3.9 g of the Intermediate 9 were used in place of 3.2 g of the Intermediate 8, and that 4.5 g of the Intermediate 3 were used in place of 5.5 g of the Intermediate 2 and as a result, 3.9 g of the pale yellow powder was obtained. By FD-MS analysis, the main peak of m / z = 691 in the case of C₅₂H₃₈N₂= 691 was recognized, therefore it was identified that the obtained was the foregoing Compound H29.

### Synthesis Example 5 (Synthesis of Compound H33)

It was carried out in a similar manner as Synthesis Example 1 except that 3.9 g of the Intermediate 10 was used in place of 3.2 g of the Intermediate 8 and as a result, 3.6 g of the pale yellow powder was obtained. By FD-MS analysis, the main peak of m / z = 793 in the case of C₆₀H₄₄N₂ = 793 was recognized, therefore it was identified that the obtained was the foregoing Compound H33.

### Synthesis Example 6 (Synthesis of Compound H41)

It was carried out in a similar manner as Synthesis Example 1 except that 3.7 g of the Intermediate 11 was used in place of 3.2 g of the Intermediate 8 and as a result, 5.1 g of the pale yellow powder was obtained. By FD-MS analysis, the main peak of m / z = 783 in the case of C₅₉H₄₆N₂ = 783 was recognized, therefore it was identified that the obtained was the foregoing Compound H41.

### Synthesis Example 7 (Synthesis of Compound H49)

It was carried out in a similar manner as Synthesis Example 1 except that 3.9 g of the Intermediate 12 was used in place of 3.2 g of the Intermediate 8 and as a result, 4.8 g of the pale yellow powder was obtained. By FD-MS analysis, the main peak of m / z = 797 in the case of C₆₀H₄₈N₂ = 797 was recognized, therefore it was identified that the obtained was the foregoing Compound H49.

### Synthesis Example 8 (Synthesis of Compound H26)

It was carried out in a similar manner as Synthesis Example 1 except that 4,0 g of the Intermediate 14 was used in place of 3.2 g of the Intermediate 8 and as a result, 5.2 g of the pale yellow powder was obtained. By FD-MS analysis, the main peak of m / z = 868 in the case of C₆₆H₄₈N₂ = 868 was recognized, therefore it was identified that the obtained was the foregoing Compound H26.

### Synthesis Example 9 (Synthesis of Compound H27)

It was carried out in a similar manner as Synthesis Example 1 except that 3.7 g of the Intermediate 15 was used in place of 3.2 g of the Intermediate 8 and as a result, 5.3 g of the pale yellow powder was obtained. By FD-MS analysis, the main peak of m / z = 842 in the case of C₆₄H₄₆N₂ = 842 was recognized, therefore it was identified that the obtained was the foregoing Compound H27.

### Synthesis Example 10 (Synthesis of Compound H30)

It was carried out in a similar manner as Synthesis Example 1 except that 3.9 g of the Intermediate 14 was used in place of 3.2g of the Intermediate 8, and that 4.5 g of the Intermediate 3 was used in place of 5.5 g of Intermediate 2 and as a result, 4.8 g of the pale yellow powder was obtained. By FD-MS analysis, the main peak of m / z = 766 in the case of C₅₆H₄₂N₂ = 766 was recognized, therefore it was identified that the obtained was the foregoing Compound H30.

### Synthesis Example 11 (Synthesis of Compound H31)

It was carried out in a similar manner as Synthesis Example 1 except that 3.9 g of the Intermediate 15 was used in place of 3.2 g of Intermediate 8, and that 4.5 g of the Intermediate 3 was used in place of 5.5 g of the Intermediate 2 and as a result, 4.1 g of the pale yellow powder was obtained. By FD-MS analysis, the main peak of m / z = 740 in the case of C₅₆H₄₀N₂ = 740 was recognized, therefore it was identified that the obtained was the foregoing Compound H31.

### Synthesis Example 12 (Synthesis of Compound H34)

It was carried out in a similar manner as Synthesis Example 1 except that 3.9 g of the Intermediate 16 was used in place of 3.2 g of the Intermediate 8 and as a result, 5.6 g of the pale yellow powder was obtained. By FD-MS analysis, the main peak of m / z = 868 in the case of C₆₆H₄₈N₂ = 868 was recognized, therefore it was identified that the obtained was the foregoing Compound H34.

### Synthesis Example 13 (Synthesis of Compound H35)

It was carried out in a similar manner as Synthesis Example 1 except that 3.9 g of the Intermediate 17 was used in place of 3.2 g of the Intermediate 8 and as a result, 5.7 g of the pale yellow powder was obtained. By FD-MS analysis, the main peak of m / z = 842 in the case of C₆₄H₄₆N₂ = 842 was recognized, therefore it was identified that the obtained was the foregoing Compound H35.

### Synthesis Example 14 (Synthesis of Compound H36)

It was carried out in a similar manner as Synthesis Example 1 except that 3.9 g of the Intermediate 18 was used in place of 3.2 g of the Intermediate 8 and as a result, 5.3 g of the pale yellow powder was obtained. By FD-MS analysis, the main peak of m / z = 944 in the case of C₇₂H₅₂N₂ = 944 was recognized, therefore it was identifed that the obtained was the foregoing Compound H36.

### Synthesis Example 15 (Synthesis of Compound H42)

It was carried out in a similar manner as Synthesis Example 1 except that 3.9 g of the Intermediate 19 was used in place of 3.2 g of the Intermediate 8 and as a result, 5.1 g of the pale yellow powder was obtained. By FD-MS analysis, the main peak of m / z = 858 in the case of C₆₄H₄₆N₂ = 858 was recognized, therefore it was identified that the obtained was the foregoing Compound H42.

### Synthesis Example 16 (Synthesis of Compound H46)

It was carried out in a similar manner as Synthesis Example 1 except that 3.9 g of the Intermediate 19 was used in place of 3.2 g of the Intermediate 8, and that 4.5 g of the Intermediate 3 was used in place of 5.5 g of the Intermediate 2 and as a result, 4.2 g of the pale yellow powder was obtained. By FD-MS analysis, the main peak of m / z = 756 in the case of C₅₇H₄₄N₂ = 756 was recognized, therefore it was identified that the obtained was the foregoing Compound H46.

### Synthesis Example 17 (Synthesis of Compound H50)

It was carried out in a similar manner as Synthesis Example 1 except that 3.9 g of the Intermediate 20 was used in place of 3.2 g of the Intermediate 8 and as a result, 5.9 g of the pale yellow powder was obtained. By FD-MS analysis, the main peak of m / z = 872 in the case of C₆₆H₅₂N₂ = 872 was recognized, therefore it was identified that the obtained was the foregoing Compound H50.

### Synthesis Example 1.8 (Synthesis of Compound H54)

It was carried out in a similar manner as Synthesis Example 1 except that 3.9 g of the Intermediate 20 in place of 3.2 g of the Intermediate 8 and as a result, 4.4 g of the pale yellow powder was obtained, and 4.5 g of Intermediate 3 in place of 5.5 g of Intermediate 2 were used. By FD-MS analysis, the main peak of m / z = 770 in the case of C₅₈H₄₆N₂ = 770 was recognized, therefore it was identified that the obtained was the above Compound H54.

### Synthesis Example 19 (Synthesis of Compound H83)

It was carried out in a similar manner as Synthesis Example 1 except that 3.9 g of the Intermediate 21 was used in place of 3.2 g of the Intermediate 8 and as a result, 4.9 g of the pale yellow powder was obtained. By FD-MS analysis, the main peak of m / z = 944 in the case of C₇₂H₅₂N₂ = 944 was recognized, therefore it was confirmed that the obtained was the foregoing Compound H83.

### Synthesis Example 20 (Synthesis of Compound H93)

Under argon gas atmospheric current, into a three neck flask with 300ml, 5.2 g of the Intermediate 23,5.5 g of the Intermediate 2, 0.26 g of Pd (PPh₃)₄, 3.18 g of sodium carbonate, 50ml of 1,2-dimethoxyethane and 30 ml of H₂O were placed and it was refluxed for 8 hours.
It was extracted by toluene and the obtained organic layer was washed by water. The organic layer was dried with the use of sodium sulfate anhydride and the solvent was removed from it by distillation. The recrystallization was carried out by using toluene / hexane, and then 6.2 g of the pale yellow powder was obtained. By FD- MS analysis, the main peak of m / z = 944 in the case of C₇₂H₅₂N₂ = 944 was recognized, therefore it was identified that the obtained was the foregoing Compound H93.

### Synthesis Example 21 (Synthesis of Compound H95)

It was carried out in a similar manner as Synthesis Example 20 except that 4.9 g of the Intermediate 24 was used in place of 5.2 g of the Intermediate 23 and that 4.5 g of the Intermediate 3 was used in place of 5.5 g of the Intermediate 2 and as a result, 5.6 g of the pale yellow powder was obtained. By FD-MS analysis, the main peak of m / z = 816 in the case of C₆₂H₄₄N₂ = 816 was recognized, therefore it was identified that the obtained was the foregoing Compound H95.

### Example 1 (Fabrication of an organic EL device)

A glass substrate (manufactured by GEOMATEC Company) of 25 mm×75 mm×1.1 mm thickness having an ITO transparent electrode was cleaned by application of ultrasonic wave in isopropyl alcohol for 5 minutes and then by exposure to ozone for 30 minutes.
The glass substrate having the transparent electrode lines which had been cleaned was attached to a substrate holder of a vacuum vapor deposition apparatus. On the surface of the cleaned substrate at the side having the transparent electrode, the following compound H232 having a thickness of 60 nm was formed so that the formed film covered the transparent electrode. The formed film of H232 worked as the hole injecting layer. Successively, a film of the foregoing compound H1 with a film thickness of 20 nm was formed over the film of H232. The formed film worked as the hole transporting layer. Further, the following compound EM1 was deposited thereby forming a film having a thickness of 40 nm. At the same time, the following amine compound D1 having styryl group as light emitting molecule was deposited with a weight ratio of EM1 : D1= 40 : 2. The formed film worked as a light emitting layer.
On the film formed above, a film of Alq having a thickness 10 nm was formed. The formed film worked as an electron injecting layer. Thereafter, Li (the source of lithium: manufactured by SAES GETTERS Company) as a reductive dopant and Alq were binary vapor deposited and an Alq:Li film (film thickness: 10 nm) was formed as the electron injecting layer (or the cathode). On the Alq:Li film, metallic aluminum was deposited to form a metal cathode and an organic El device was fabricated.
The results of Measuring vapor deposition temperature for the hole transporting material on the fabrication process of the organic EL device, of observing the appearance of the vapor deposition board after the deposition, and the glass transition temperature (Tg) are shown in Table 1.Further, current efficiency was measured on the fabricated organic EL device and the emitted color was observed. The luminance was measured by using CS1000 manufactured by MINOLTA and then the current efficiency at 10mA/cm²was worked out. The result from measuring the half-lifetime of the emission at 2000 nit of the initial luminance, room temperature and driving it by applying the constant direct current was shown in Table 1.
In addition, Tg was the numeric datum on 2nd heating under the following condition by using DSC "Pyris1" of PerkinElmer, Inc.:(i) a test sample was heated from 30 °C to maximum temperature at 10 °C/minute, (ii) it was kept for 3 minutes at the maximum temperature, (iii) it was cooled from the maximum temperature to -50 °C at 200 °C/minute, (iv) it was kept for 10 minutes at -50 °C, and (v) it was heated from -50 °C to the maximum temperature at 10 °C/minute; the maximum temperature was a melting point derived from Tg-DTA measurement plus about 30 °C and it was properly adjusted when the decomposition temperature was close to the melting point.

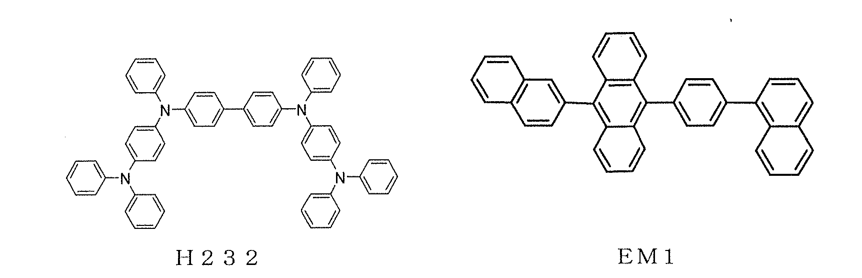

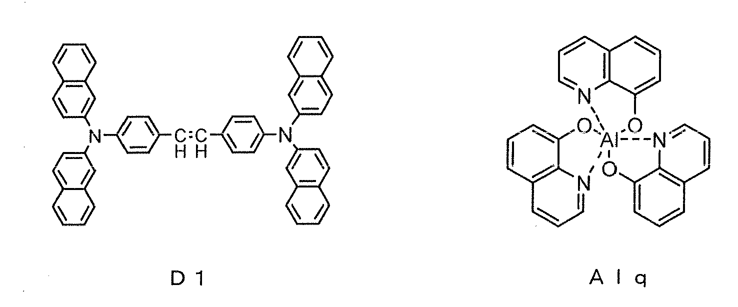

### Examples 2 to 21 (Fabrication of organic EL devices)

Organic EL devices were fabricated similarly as Example 1 except that Compounds described in Table 1 as the hole transporting material was used in place of Compound H1.
The results of measuring vapor deposition temperature for the hole transporting material on the fabrication process of the organic EL device, of observing the appearance of the vapor deposition board after the deposition, and the glass transition temperature (Tg) are shown in Table 1. Further, current efficiency was measured on the fabricated organic EL device and the emitted color was observed, and also the result from measuring the half-lifetime of the light emission at 2000 nit of the initial luminance, room temperature and driving it by applying the constant direct current was shown in Table 1.

### Comparative Examples 1 to 4

Organic EL devices were fabricated similarly as Example 1 except that Comparative Compound 1 (Comparative Example 1), Comparative Compound 2 (Comparative Example 2), Comparative Compound 3 (Comparative Example 3) and Comparative Compound 4 (Comparative Example 4) as a hole transporting material were used in place of Compound H1.
The results of measuring vapor deposition temperature for the hole transporting material on the fabrication process of the organic EL device, of observing the appearance of the vapor deposition board after the deposition, and the glass transition temperature (Tg) are shown in Table 1.
Further, current efficiency was measured on the fabricated organic EL device and the emitted color was observed, and also the result from measuring the half-lifetime of the emission at 2000 nit of the initial luminance, room temperature and driving it by applying the constant direct current was shown in Table 1.

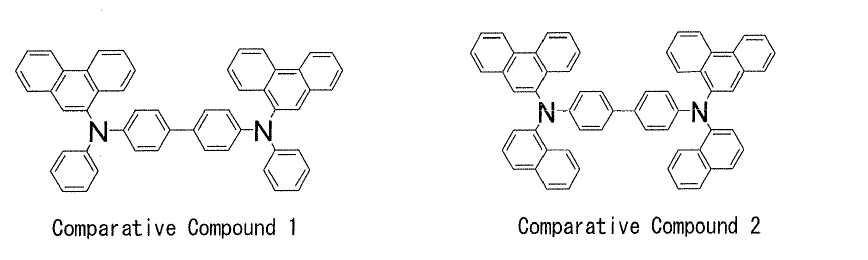

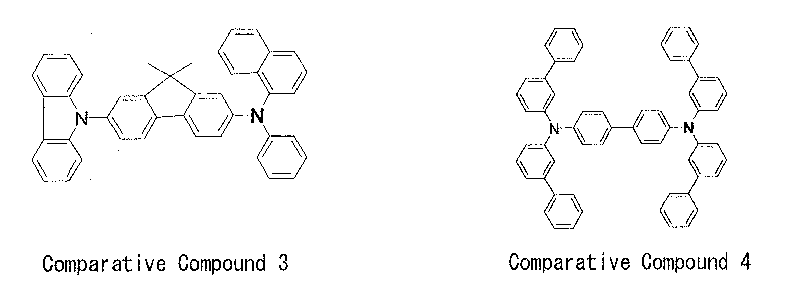

**Table 1**

| | Hole transporting material | Current Efficiency | Color of Light Emission | Half Lifetime | Vapor Deposition Temp. | Tg | Deposition Board |
|---|---|---|---|---|---|---|---|
| | | (cd/A) | | (hours) | (°C) | (°C) | |
| Ex. 1 | H1 | 6.0 | Blue | 2700 | 350 | 138 | No Change |
| Ex. 2 | H5 | 6.2 | Blue | 2200 | 330 | 124 | No Change |
| Ex. 3 | H25 | 7.0 | Blue | 2900 | 360 | 128 | No Change |
| Ex. 4 | H29 | 6.4 | Blue | 2400 | 330 | 114 | No Change |
| Ex. 5 | H33 | 6.3 | Blue | 3100 | 350 | 125 | No Change |
| Ex. 6 | H41 | 6.3 | Blue | 2600 | 340 | 128 | No Change |
| Ex. 7 | H49 | 6.6 | Blue | 2700 | 350 | 134 | No Change |
| Ex. 8 | H26 | 7.2 | Blue | 2,300 | 380 | 142 | No Change |
| Ex. 9 | H27 | 7.1 | Blue | 2,400 | 370 | 144 | No Change |
| Ex. 10 | H30 | 7.2 | Blue | 2,200 | 350 | 126 | No Change |
| Ex. 11 | H31 | 7.3 | Blue | 2,400 | 340 | 128 | No Change |
| Ex. 12 | H34 | 6.8 | Blue | 3,400 | 380 | 128 | No Change |
| Ex. 13 | H35 | 6.6 | Blue | 3,200 | 370 | 127 | No Change |
| Ex. 14 | H36 | 6.8 | Blue | 3,400 | 390 | 123 | No Change |
| Ex. 15 | H42 | 6.9 | Blue | 2,100 | 380 | 145 | No Change |
| Ex. 16 | H46 | 6.2 | Blue | 2,300 | 350 | 127 | No Change |
| Ex. 17 | H50 | 6.7 | Blue | 2,000 | 380 | 146 | No Change |
| Ex. 18 | H54 | 6.0 | Blue | 2,300 | 360 | 129 | No Change |
| Ex. 19 | H83 | 7.1 | Blue | 2,800 | 390 | 152 | No Change |
| Ex. 20 | H93 | 6.1 | Blue | 2,500 | 390 | 138 | No Change |
| Ex. 21 | H95 | 6.3 | Blue | 2,300 | 380 | 137 | No Change |
| Co. Ex.1 | Co. Comp. 1 | 5.6 | Blue | 1600 | 400 | 146 | Changed |
| Co. Ex.2 | Co. Comp. 2 | 5.2 | Blue | 1200 | 420 | 165 | Changed |
| Co. Ex.3 | Co. Comp. 3 | 6.6 | Blue | 900 | 280 | 109 | No Change |
| Co. Ex.4 | Co. Comp. 4 | 5.5 | Blue | 1300 | 380 | 95.8 | No Change |

In Table 1, "Ex.", "Co.", "Comp." and "Temp." are respectively abbreviations of "Example", "Comparative", "Compound" and "Temperature". Further, "No Change" and "Changed" each respectively means "No Change in color at residue." and "Changed in color at residue." in this order.

### Example 22 (Fabrication of an organic EL device)

An organic EL device was fabricated similarly as Example 1 except that the following arylamine compound D2 was used in place of Amine compound D1 having a styryl group. Me represents a methyl group.
The current efficiency measured on the fabricated organic EL device was 5.2 cd/A and the emitted color was blue. Further, the half-lifetime of the emission at 2000 nit of the initial luminance, room temperature and driving it by feeding the constant direct current was 2800 hours.

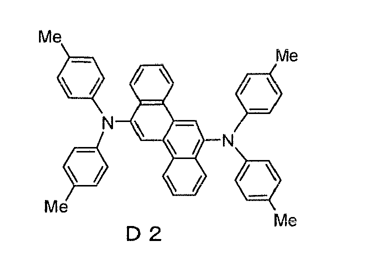

### Comparative Example 5

An organic EL device was fabricated similarly as Example 22 except that the above Comparative compound 1 as the hole transporting material was used in place of Compound H1.
The current efficiency measured on the fabricated organic EL device was 4.8 cd/A and the emitted color was blue. Further, the half-lifetime of the emission at 2000 nit of the initial luminance, room temperature and driving it by feeding the constant direct current was 1500 hours.

As shown from the above results, when the aromatic amine derivatives of the present invention are used for a hole transporting material of an organic EL device, it is possible to emit light on the same level of current efficiency with known materials, and also they have an asymmetric structure so that they could be deposited at low sublimation temperature. Therefore, the decomposition thereof on vapor deposition was controlled, and uneven deposition is rarely caused. Accordingly, the aromatic amine derivatives are distinctly effective on lasting the lifetime of the organic EL device longer.

### Industrial Applicability

As explained above in details, since the aromatic amine derivatives of the present invention have steric hindrance so that interaction between the molecules is small, crystallization thereof is controlled and the success ratio on an organic EL device production is improved. Further, since it could be deposited at low sublimation temperature so that decomposition of the molecules on vapor deposition was controlled, an organic EL device obtained by using the compound has an advantage of a longer lifetime.

## Claims

1. An aromatic amine derivative represented by the following general formula (1):
A - L - B (1)
wherein, L represents an interbonding group consisting of a substituted or unsubstituted arylene group having 5 to 50 ring atoms, or an interbonding group derived from bonding a plural number of a substituted or unsubstituted arylene group having 5 to 50 ring atoms with a single bond, an oxygen atom, a sulfur atom, a nitrogen atom or a saturated or unsaturated bivalent aliphatic hydrocarbon group having 1 to 20 carbon atoms;
A represents a diarylamino group expressed by a following general formula (2); 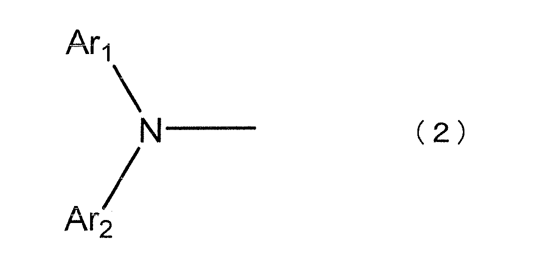
B represents a diarylamino group expressed by a following general formula (3), however, A is not the same as B; 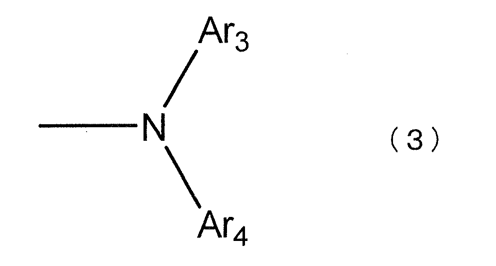
wherein Ar₂ to Ar₄ in the general formulae (2) and (3) each independently represents a substituted or unsubstituted aryl group having 5 to 50 ring atoms;
Ar₁ in the general formula (2) represents any of following general formulae (4) to (9): 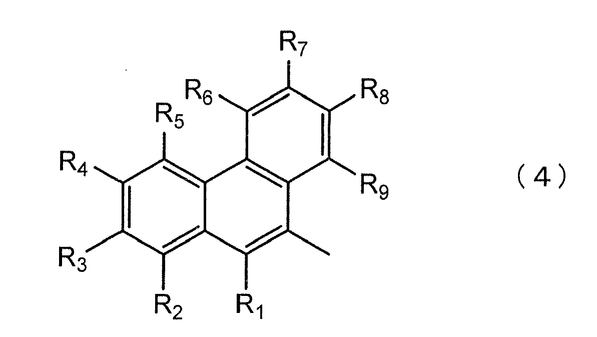 wherein R₁ to R₉ each independently represents a hydrogen atom, an aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 ring atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted with a substituted or unsubstituted aryl group having 5 to 50 ring atoms, a halogen atom, a cyano group, a nitro group, a hydroxyl group or a carboxyl group; 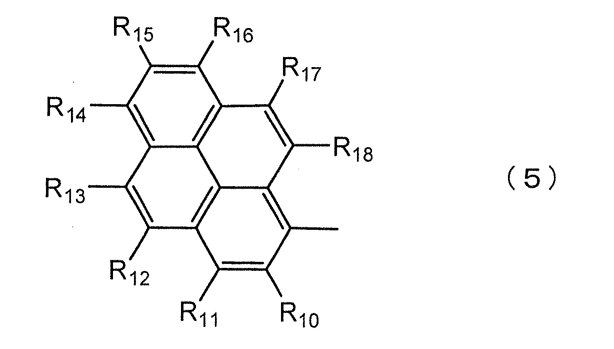 wherein R₁₀ to R₁₈ each independently is the same as each of R₁ to R₉ in the general formula (4); 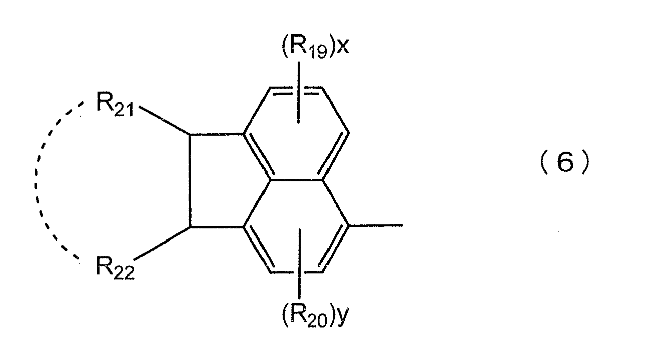 wherein R₁₉ to R₂₂ each independently is the same as each of R₁ to R₉ in the general formula (4);
x represents an integer of 0 to 3, y represents an integer of 0 to 2. R₂₁ and R₂₂ may bond each other to form a ring structure, 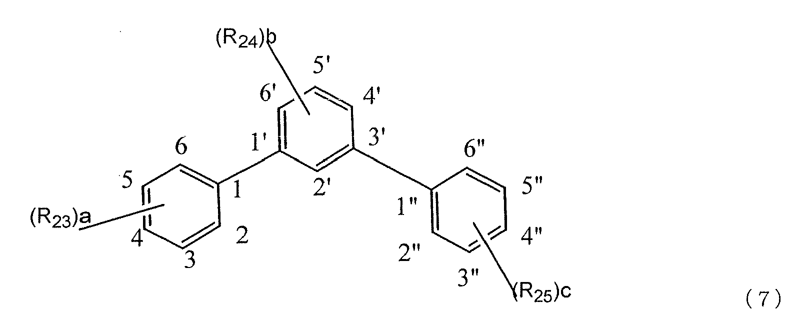 which corresponds to a m-terphenyl group having an interbonding hand obtained by removing a hydrogen atom at any one of 2 to 6, 4' to 6 and 2" to 6" positions from it, and R₂₃ to R₂₅ each independently is the same as each R₁ to R₉ in the general formula (4);
a and c each independently represents an integer of 0 to 5, and b represents an integer of 0 to 4; 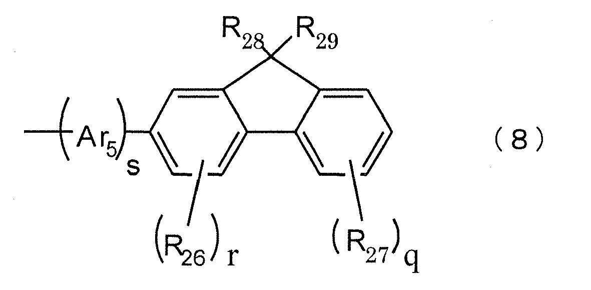 wherein Ar5 represents a substituted or unsubstituted arylene group or polyarylene group both having 5 to 50 ring atoms, or a bivalent group consisting of a substituted or unsubstituted heterocyclic group diary heterocyclic group both having 5 to 50 ring atoms;
R₂₆ to R₂₉ each independently is the same as each R₁ to R₉ in the general formula (4); s, q and r each independently represents an integer of 0 to 2, and
a couple of R₂₈ and R₂₉ may bond each other to form a ring structure.

2. The aromatic amine derivative according to Claim 1, wherein B in the aforementioned general formula (1) represents a diarylamino group represented by a following general formula (9): 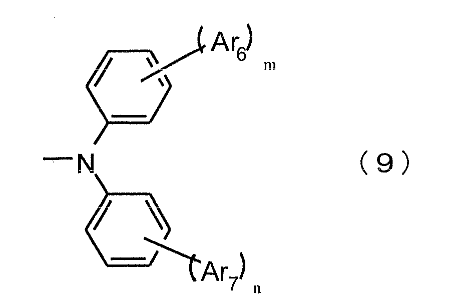 wherein Ar₆ and Ar₇ each independently represents a substituted or unsubstituted aryl group having 5 to 50 ring atoms;
m represents an integer of 1 to 5 and n represents an integer of 0 to 5.

3. The aromatic amine derivative according to Claim 1, wherein B in the aforementioned general formula (1) represents a diarylamino group represented by the following general formula (10): 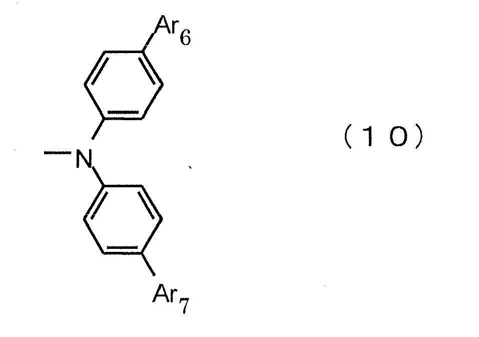 wherein Ar₆ and Ar₇ each independently represents a substituted or unsubstituted aryl group having 5 to 50 ring atoms.

4. The aromatic amine derivative according to Claim 1, wherein Ar₁ is different from any of Ar₂ to Ar₄ in the aforementioned general formula (1).

5. The aromatic amine derivative according to Claim 1, wherein at least one of Ar₂ to Ar₄ represents a substituted or unsubstituted condensed ring having 10 to 50 ring atoms in the aforementioned general formula (1).

6. The aromatic amine derivative according to Claims 1 to 5, wherein the derivative is a material for an organic electroluminescence device.

7. The aromatic amine derivative according to Claims 1 to 5, wherein the derivative is a hole transporting material for an organic electroluminescence device.

8. An organic electroluminescence device which comprises at least one organic thin film layer comprising a light emitting layer sandwiched between a pair of electrodes consisting of an anode and a cathode, wherein at least one of the organic thin film layers comprises the aromatic amine derivative according to Claim 1 singly or as its mixture component.

9. The organic electroluminescence device according to Claim 8, wherein said organic thin film layer comprises a hole transporting layer, and the hole transporting layer comprises the aromatic amine derivative according to Claim 1 singly or as its mixture component.

10. The organic electroluminescence device according to Claim 8, wherein said light emitting layer comprises at least one of an arylamine compound or a styrylamine compound.

11. The organic electroluminescence device according to Claims 8 to 10, which emits bluish light.
